(19) Europäisches Patentamt
European Patent Office
Office européen des brevets

(11) Publication number: **0 044 185**
**A2**

# (12) EUROPEAN PATENT APPLICATION

(21) Application number: 81303077.2

(22) Date of filing: 06.07.81

(51) Int. Cl.³: C 07 D 413/04
A 01 N 43/80

(30) Priority: 07.07.80 US 166011

(43) Date of publication of application: 20.01.82 Bulletin 82/3

(84) Designated Contracting States: AT BE CH DE FR GB IT LI LU NL SE

(71) Applicant: ELI LILLY AND COMPANY
307, East McCarty Street
Indianapolis Indiana 46206(US)

(72) Inventor: Burow, Kenneth Wayne, Jr.
5449 E. 62nd Street
Indianapolis Indiana 46220(US)

(72) Inventor: St. Clair, Roger Lee
R.R. 1 Box 80
Greenfield Indiana 46140(US)

(74) Representative: Crowther, Terence Roger et al,
Erl Wood Manor
Windlesham Surrey GU20 6PH(GB)

## (54) Imidazolidinone derivatives.

(57) Isoxazolylimidazolidinones of the formula I

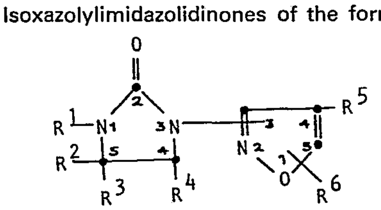

I

wherein:

$R^1$ is $C_1-C_6$ alkyl;

$R^2$ is hydrogen, or taken together with $R^4$ is a double bond;

$R^3$ is hydrogen or $C_1-C_6$ alkyl;

$R^4$ is hydroxy, $C_1-C_6$ alkanoyloxy, or taken together with $R^2$ is a double bond;

$R^5$ is hydrogen, halo, cyano, nitro, or $C_1-C_6$ alkyl; and

$R^6$ is hydrogen, $C_1-C_6$ alkyl, trifluoromethyl, $C_1-C_3$ alkoxy-$C_1-C_6$ alkyl, $C_3-C_7$ cycloalkyl or $C_1-C_3$ alkyl substituted $C_3-C_7$ cycloalkyl are useful as herbicides both terrestrial and aquatic.

EP 0 044 185 A2

# IMIDAZOLIDINONE DERIVATIVES

This invention relates to novel isoxazolylimidazolidinone derivatives which have herbicidal activity against both terrestrial and aquatic weeds, to herbicidal formulations thereof, and to a process for their preparation.

A number of substituted imidazolidinones are known which are said to display selective herbicidal activity. Krenzer, in U.S. Patent No. 4,093,443, describes certain 2-thiadiazolylimidazolidinones which are allegedly useful as herbicides. Similar thiadiazolylimidazolidinone compounds are disclosed by Krenzer in U.S. Patent Nos. 3,904,640, 3,944,409, 3,963,473, 3,964,895, 3,984,228, 3,990,882, 4,018,787, 4,021,439, 4,028,375, 4,063,924, and 4,097,486.

Imidazolidinones bearing an isoxazolyl moiety are heretofore unknown. The compounds provided by the present invention are imidazolidinone derivatives which require an isoxazolyl substituent at the 3 or 5-position.

According to the present invention there is provided an isoxazolylimidazolidinone of the formula I

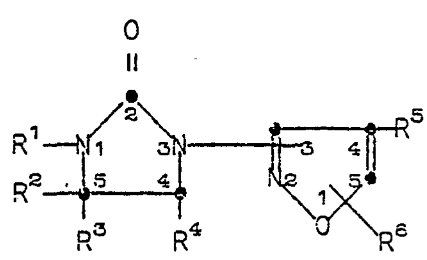

I

wherein:

$R^1$ is $C_1$-$C_6$ alkyl;

$R^2$ is hydrogen, or taken together with $R^4$ is a double bond;

$R^3$ is hydrogen or $C_1$-$C_6$ alkyl;

$R^4$ is hydroxy, $C_1$-$C_6$ alkanoyloxy, or taken together with $R^2$ is a double bond;

$R^5$ is hydrogen, halo, cyano, nitro, or $C_1$-$C_6$ alkyl; and

$R^6$ is hydrogen, $C_1$-$C_6$ alkyl, trifluoromethyl, $C_1$-$C_3$ alkoxy-$C_1$-$C_6$ alkyl, $C_3$-$C_7$ cycloalkyl, or $C_1$-$C_3$ alkyl substituted $C_3$-$C_7$ cycloalkyl.

There is also provided a herbicidal formulation which comprises as active ingredient from 1.0 to 90.0% by weight of an isoxazolylimidazolidinone of the formula I as before defined associated with at least one carrier or diluent therefor.

There is also provided a process for preparing an isoxazolylimidazolidinone of the formula I as before described which comprises a) cyclising a (dialkoxy)-ethyl isoxazolylurea of the formula II

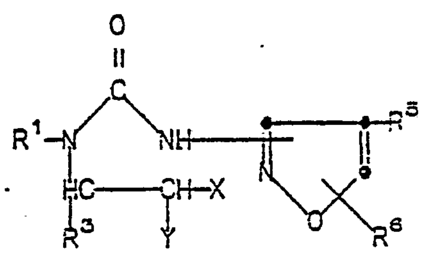

II

wherein $R^1$, $R^3$, $R^5$ and $R^6$ are as above described and X and Y independently are $C_1$-$C_6$ alkoxy to obtain a compound of formula I in which $R^4$ is hydroxy, b) dehydrating a compound of formula I, in which $R^4$ is hydroxy, to obtain a compound of formula I in which $R^4$ together with $R_2$ signify a double bond, or c) acylating a compound of formula I, in which $R^4$ is hydroxy, to obtain a compound of formula I in which $R^4$ is $C_1$-$C_6$ alkanoyloxy.

There is also provided a method of inhibiting the growth of weeds which comprises applying to the

Typical "$C_3$-$C_7$ cycloalkyl" groups are cyclopropyl, cyclobutyl, cyclopentyl, cyclohexyl and cycloheptyl. A "$C_1$-$C_3$ alkyl substituted $C_3$-$C_7$ cycloalkyl" group is one of the recited cycloalkyl groups which is monosubstituted with a $C_1$-$C_3$ alkyl group such as methyl, ethyl and propyl. It is preferred that the $C_1$-$C_3$ alkyl substituent group be attached at the 1-position of the cycloalkyl group. Typical of such preferred groups are 1-methylcyclopentyl, 1-methylcyclohexyl, 1-ethylcycloheptyl, 1-n-propylcyclobutyl and the like.

The term "halo" as used herein refers to fluoro, chloro, bromo and iodo. Preferred halo groups include chloro and bromo.

Most of the isoxazolylimidazolidinone derivatives provided by this invention are prepared by cyclization of a suitably substituted 3 or 5-isoxazyolylurea of the formula II

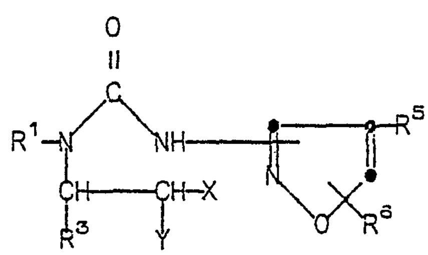 II

wherein $R^1$, $R^3$, $R^5$ and $R^6$ are as defined above, and X and Y are $C_1$-$C_6$ alkoxy such as methoxy, ethoxy, propoxy or the like.

The cyclization of the di-alkoxy isoxazolylurea intermediate can be accomplished by simply heating the intermediate in the presence of an acid. Commonly used acids include mineral acids such as hydrochloric,

hydrobromic, sulfuric, phosphoric, nitric, and related acids. Organic acids such as acetic acid can also be utilized if desired. The amount of acid commonly employed to effect the cyclization is about an equimolar amount or an excess, and routinely the reaction is simply carried out in a dilute aqueous acid solution having an acid concentration of about 0.5 to about 5 percent by weight. If desired, the cyclization reaction can be carried out in a solvent medium other than water, such as dioxane, dimethylsulfoxide, dimethylformamide, and the like, with an equimolar or excess amount of suitable acid added. The cyclization reaction generally is substantially complete within about 10 to about 90 minutes when carried out at an elevated temperature of about 40 to about 100°C. The reaction provides an isoxazolyl-4-hydroxyimidazolidinone (i.e. an imidazolidinone wherein $R^4$ is hydroxy) which is readily isolated by cooling the reaction mixture, for instance to 0° to about 5°C., and then collecting the precipitate. The precipitated isoxazolyl-4-hydroxyimidazolidinone can be further purified if desired by conventional means, including chromatography and crystallization from common solvents such as ethanol, acetone, dioxane, water and the like.

Examples of isoxazolyl-4-hydroxyimidazolidinones which are readily prepared according to the above described cyclization process include the following:

3-(4-cyano-3-isoxazolyl)-4-hydroxy-1-ethyl-2-imidazolidinone;

3-(4,5-diethyl-3-isoxazolyl)-4-hydroxy-1-methyl-2-imidazolidinone;

3-(4-*iso*propyl-3-isoxazolyl)-4-hydroxy-1-*n*-butyl-2-imidazolidinone;

3-(5-*iso*propyl-3-isoxazolyl)-4-hydroxy-1-*n*-butyl-2-imidazolidinone;

3-(3-trifluoromethyl-5-isoxazolyl)-4-hydroxy-1-methyl-2-imidazolidinone;

3-(3-ethyl-4-nitro-5-isoxazolyl)-4-hydroxy-1-ethyl-2-imidazolidinone;

3-(5-cyclohexyl-3-isoxazolyl)-4-hydroxy-1-methyl-2-imidazolidinone;

3-(3-*tert*.-butyl-4-cyano-5-isoxazolyl)-4-hydroxy-1-*n*-propyl-2-imidazolidinone;

3-[5-(1-ethyl-1-methylpropyl)-3-isoxazolyl]-4-hydroxy-1-methyl-2-imidazolidinone;

3-[3-(1-ethyl-1-methylpropyl)-5-isoxzaolyl]-4-hydroxy-1-ethyl-2-imidazolidinone;

3-(3-methoxymethyl-4-bromo-5-isoxazolyl)-4-hydroxy-1-ethyl-2-imidazolidinone; and

3-(4-cyano-5-*tert*.-butyl-3-isoxazolyl)-4-hydroxy-1-*n*-hexyl-2-imidazolidinone.

The isoxazolyl-4-hydroxyimidazolidinones thus described are useful not only as herbicides, but also as intermediates in the synthesis of other imidazolidinones of the invention. For example, the unsaturated imidazolinones of the invention, i.e. compounds of the above formula wherein $R^2$ and $R^4$ together form a double bond, are preferably prepared by dehydrating a 4-hydroxyimidazolidinone according to the following sequence:

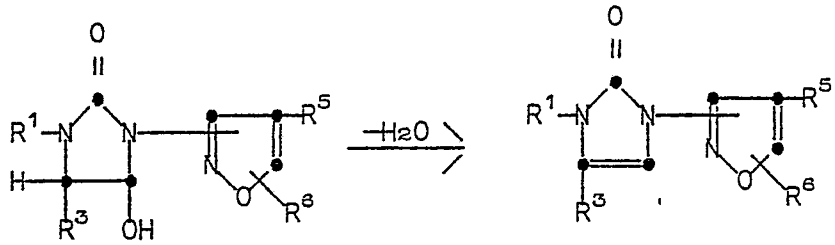

Such dehydration can be effected by reacting the 4-hydroxyimidazolidinone with an acid such as a mineral acid, or preferably with an equimolar amount or slight excess of a halogenating agent such as thionyl chloride. For example, reaction of a compound such as 3-(5-tert.-butyl-3-isoxazolyl)-4-hydroxy-1-ethyl-2-imidazolidinone with about an equimolar amount of thionyl chloride in a solvent such as chloroform or methylene chloride for about 1 to about 24 hours at a temperature of about 0 to about 50°C. provides the corresponding unsaturated imidazolinone, namely 3-(5-tert.-butyl-3-isoxazolyl)-1-ethyl-2-imidazolinone. The product can be recovered by simply removing the reaction solvent, for instance by evaporation, and further purification can be achieved if desired by routine procedures such as crystallization from common solvents such acetone or diethyl ether.

The isoxazolyl-4-hydroxy-imidazolidinones of this invention are also useful in the preparation of those imidazolidinones of the above general formula wherein $R^4$ is $C_1$-$C_6$ alkanoyloxy. For example, the isoxazolyl-4-hydroxy-imidazolidinones of the invention are readily acylated with an acylating agent such as an acid halide. Commonly used acylating agents include acetyl chloride, butyryl iodide, hexanoyl bromide, and

the like. Other acylating agents commonly employed include anhydrides such as acetic anhydride, acetic formic anhydride, propanoic formic anhydride, and the like.

The acylation of an isoxazolyl-4-hydroxy-imidazolidinone can be accomplished by reacting about equimolar quantities of a 4-hydroxyimidazolidinone and an acylating agent. An excess of acylating agent can be employed if desired. The reaction typically is conducted in a solvent such as benzene, acetone, dichloromethane, chloroform, or the like, and routinely is carried out in the presence of a base to act as an acid scavenger. Commonly used bases include triethylamine, pyridine, sodium carbonate, and the like. The acylation reaction is generally substantially complete after about 2 to about 24 hours when carried out at a temperature of about -20 to about 80°C. The acylated product, namely an isoxazolylimidazolidinone of the above formula wherein $R^4$ is $C_1$-$C_6$ alkanoyloxy, can be isolated by simply removing the reaction solvent, for instance by evaporation under reduced pressure. The acylated imidazolidinone can be purified if needed by conventional means, including washing with dilute acid and dilute base, chromatography, crystallization, and the like.

Typical compounds comprehended by this invention which are conveniently prepared by acylation or dehydration of a 4-hydroxy-imidazolidinone include the following:

3-(5-*tert*.-butyl-3-isoxazolyl)-4-acetoxy-1-*n*-propyl-2-imidazolidinone;

3-(5-methoxymethyl-3-isoxazolyl)-4-acetoxy-1-(1,1-dimethylpropyl)-2-imidazolidinone;

3-[3-(2-methoxypentyl)-5-isoxazolyl]-4-*n*-butyroxy-5-ethyl-1-methyl-2-imidazolidinone;

3-(5-*tert*.-butyl-3-isoxazolyl)-4-propionoxy-1-(1,1-dimethylbutyl)-2-imidazolidinone;

3-(4-cyano-3-isoxazolyl)-4-acetoxy-1-*iso*propyl-2-imidazolidinone;

3-(5-cycloheptyl-3-isoxazolyl)-4-acetoxy-1-ethyl-2-imidazolidinone;

3-(5-*iso*butyl-3-isoxazolyl)-4-acetoxy-1-methyl-2-imidazolidinone;

3-(4-nitro-5-*sec*.-butyl-3-isoxazolyl)-4-propionoxy-5-methyl-1-ethyl-2-imidazolidinone;

3-(5-isoxazolyl)-4-acetoxy-1-*n*-butyl-2-imidazolidinone;

3-(3-trifluoromethyl-5-isoxazolyl)-4-*n*-butyroxy-1-ethyl-2-imidazolidinone;

3-(5-methoxymethyl-3-isoxazolyl)-4-formyloxy-1-*tert*.-butyl-2-imidazolidinone;

3-(4-chloro-5-ethyl-3-isoxazolyl)-4-propionoxy-5-ethyl-1-*n*-propyl-2-imidazolidinone;

3-(5-*iso*butyl-3-isoxazolyl)-4-acetoxy-1-(2-methylpentyl)-2-imidazolidinone;

3-(3-methyl-5-isoxazolyl)-4-*n*-butyroxy-5-ethyl-1-(1,1-dimethylpropyl)-2-imidazolidinone;

3-(5-*tert*.-butyl-3-isoxazolyl)-1-ethyl-2-imidazolinone;

3-(4,5-diethyl-3-isoxazolyl)-1-methyl-2-imidazolinone;

3-[3-(1-ethylcyclopentyl)-5-isoxazolyl)-1-methyl-2-imidazolinone;

3-(5-methoxymethyl-3-isoxazolyl)-1-methyl-2-imidazolinone; and

3-(4-bromo-5-isopropoxymethyl-3-isoxazolyl)-5-methyl-1-(1,1-dimethylpropyl)-2-imidazolinone.

As pointed out earlier, the isoxazolylimidazolidinone derivatives of this invention require, as an initial starting material, an isoxazolylurea of the formula II

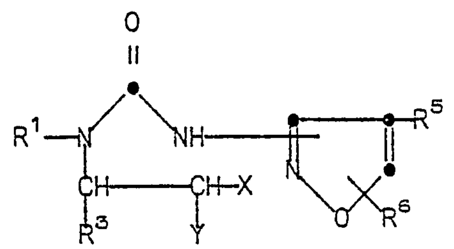

II

wherein X and Y are $C_1$-$C_6$ alkoxy groups such as methoxy, ethoxy or the like. The isoxazolylurea intermediates can be prepared by any of several procedures. In a typical procedure, a 3 or 5-aminoisoxazole is reacted with a haloformate such as phenyl chloroformate to give an isoxazolyl carbamate, which when reacted with a suitably substituted ethylamine gives the isoxazolylurea of the invention. Such procedure can be illustrated by the following sequence:

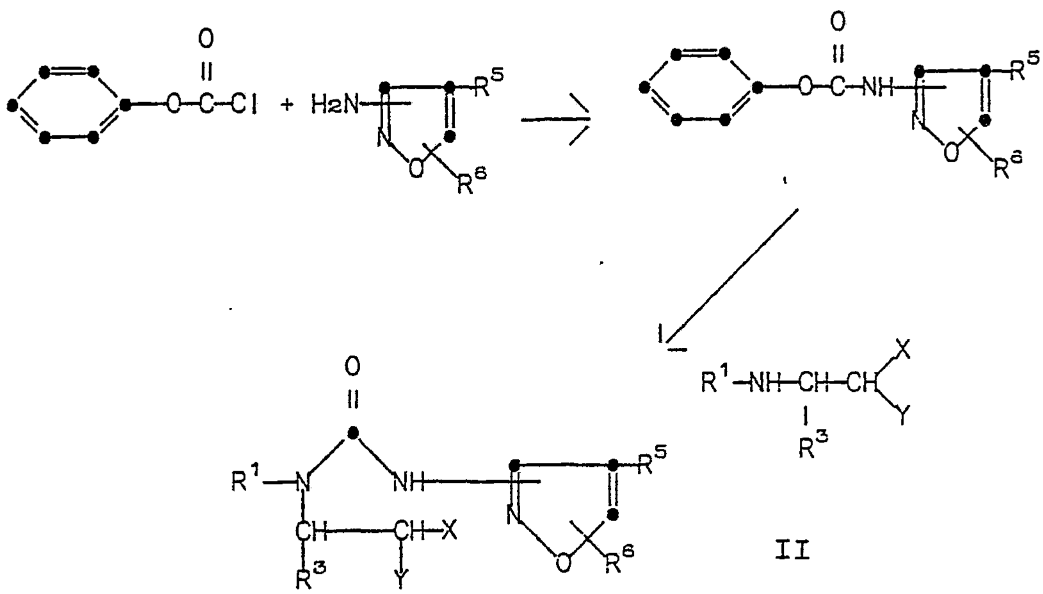

II

The 3 or 5-aminoisoxazoles, wherein $R^5$ and $R^6$ are as defined above, are known in the art or are available by art known methods. Similarly, the dialkoxy ethylamines of the formula $R^1NHCH(R^3)CHXY$ are well known in the art and some are commercially available.

The reaction of a haloformate such as phenyl chloroformate and a 3 or 5-aminoisoxazole generally is carried out by combining an excess of haloformate with the isoxazole in an organic solvent such as pyridine or triethylamine, and stirring the mixture for about 1 to about 24 hours at a temperature of about 0 to about 30°C. The product of the reaction, an isoxazolyl carbamate, is generally isolated by acidifying the reaction mixture, for instance by the addition of a mineral acid such as hydrochloric acid or sulfuric acid, and collecting by filtration the precipitate which forms. The carbamate normally needs no further

purification, but if desired it can be crystallized from common solvents such as benzene, acetone, ethyl acetate and the like.

The isoxazolyl carbamate thus formed is next reacted with an N-alkyl-2,2-dialkoxy ethylamine derivative. Typical ethylamines commonly employed include N-methyl-2,2-dimethoxyethylamine, N-ethyl-2,2-diethoxyethylamine, N-*iso*butyl-1-methyl-2-ethoxy-2-methoxyethylamine, N-(1,1-dimethylpropyl)-1-*n*-hexyl-2-methoxy-2-hexyloxyethylamine, and the like.

The isoxazolyl carbamate and the ethylamine generally are combined in a mutual organic solvent such as toluene or benzene. The reactants can be utilized in about equimolar amounts, or if desired the ethylamine derivative can be employed in excess. The reaction routinely is substantially complete within about 2 to about 5 hours when carried out at a temperature of about 50 to about 100°C. Isolation of the product, an isoxazolylurea intermediate of the invention, usually is accomplished by simply removing the reaction solvent, for instance by evaporation under reduced pressure. The isoxazolylurea can be further purified if needed by routine methods, including recrystallization and chromatography.

An alternative procedure for preparing the isoxazolylurea intermediates of the invention comprises reacting a suitably substituted N-alkyl-2,2-dialkoxy ethylamine derivative with a 3 or 5-isoxazolyl isocyanate. The isoxazolyl isocyanate is conveniently prepared by reacting a 3 or 5-aminoisoxazole with phosgene

in the presence of an acid such as hydrochloric acid to give the corresponding 3 or 5-chlorocarbonylamino-isoxazole. The latter compound undergoes dehydrohalogenation in situ to provide the corresponding isoxazolyl isocyanate. The reaction of the isocyanate with an ethylamine derivative is illustrated by the following sequence:

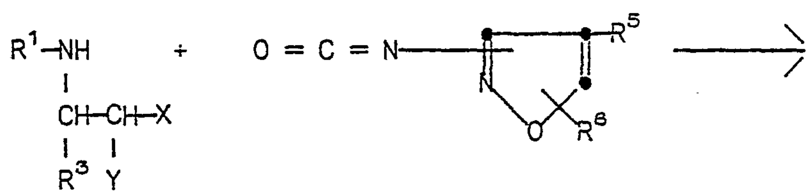

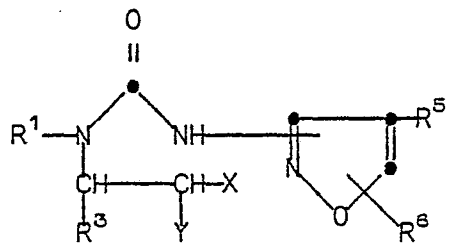

II

wherein $R^1$, $R^3$, $R^5$, $R^6$, X and Y are all as heretofore defined. The reaction of an ethylamine with an isoxazolyl isocyanate generally is carried out by combining approximately equimolar quantities of the reactants in a suitable solvent such as benzene or toluene. The reaction normally is complete within about 2 to about 5 hours when carried out at a temperature of about 50 to about 100°C. Isolation of the product generally is achieved by simply removing the reaction solvent by evaporation under reduced pressure. Further purification, if needed, can be accomplished by crystallization, chromatography or similar conventional methods.

The preparation of typical isoxazolylurea intermediates and isoxazolylimidazolidinone derivatives is illustrated by the following working examples. The examples are representative only and are not intended to be limiting in any respect and should not be so construed.

Example 1

3-(5-*tert*.-butyl-3-isoxazolyl)-4-hydroxy-1-methyl-2-imidazolidinone.

A. Preparation of 5-*tert*.-butyl-3-(N-phenoxycarbonylamino)isoxazole.

A solution of 30 g. of a mixture comprised of 80% 5-*tert*.-butyl-3-aminoisoxazole and 20% 5-amino-3-*tert*.-butylisoxazole in 500 ml. of pyridine was cooled to 0°C. in an ice bath and stirred. To the cold stirred reaction mixture was added dropwise over thirty minutes 35 g. of phenyl chloroformate. Following the addition, the reaction mixture was stirred for one hour at 0°C. and then poured over ice. The aqueous mixture was acidified to pH 2.0 with concentrated hydrochloric acid. The aqueous acid mixture was filtered to collect the precipitate which had formed. The precipitate was air dried and shown to consist of 5-*tert*.-butyl-3-(N-phenoxycarbonylamino)-isoxazole.

M.P. 94-97°C. Yield 36 g.

B. Preparation of 1-(5-*tert*.-butyl-3-isoxazolyl)-3-[2,2-(dimethoxy)ethyl]-3-methylurea.

To a solution of 36 g. of 5-*tert*.-butyl-3-(N-phenoxycarbonylamino)-isoxazole in 300 ml. of toluene were added in one portion 20 g. of N-methyl-(2,2-dimethoxy)ethylamine. The reaction mixture was heated at reflux for three hours, and then was cooled and concentrated to an oil by evaporation of the solvent under reduced pressure. The oil thus formed was purified by chromatography over silica gel, eluting with diethyl ether. The appropriate fractions were collected, and the solvent was removed therefrom to afford 27.2 g. of 1-(5-*tert*.-butyl-3-isoxazolyl)-3-[2,2-(dimethoxy)ethyl]-3-methylurea.

M.P. 78-79°C.

Analysis calculated for $C_{13}H_{23}N_3O_4$

Theory: C, 54.72; H, 8.12; N, 14.73.

Found: C, 54.43; H, 7.95; N, 14.64.

C. 3-(5-*tert*.-butyl-3-isoxazolyl)-4-hydroxy-1-methyl-2-imidazolidinone.

A solution of 11 g. of 1-(5-*tert*.-butyl-3-isoxazolyl)-3-[2,2-(dimethoxy)ethyl]-3-methylurea in 100 ml. of 0.5 N hydrochloric acid was heated at 50°C. for thirty minutes. The reaction mixture was then cooled to room temperature and added to 200 g. of ice. The solid precipitate which formed was collected by filtration and recrystallized from ethyl acetate to provide 8.9 g. of 3-(5-*tert*.-butyl-3-isoxazolyl)-4-hydroxy-1-methyl-2-imidazolidinone.

M.P. 176-177°C.

Analysis calculated for $C_{11}H_{17}N_3O_3$

Theory: C, 55.22; H, 7.16; N, 17.56.

Found: C, 55.51; H, 6.88; N, 17.35.

## Example 1A

Example 1 was repeated on a larger scale as follows:

A. A solution of 560.8 g. of pure 5-*tert*.-butyl-3-aminoisoxazole in 10 liters of pyridine was cooled to 0°C. and stirred while 688.9 g. (553.7 ml.) of phenyl chloroformate were added dropwise over one hour. The reaction mixture was stirred at 0°C. for two hours following the addition, and then was warmed to room temperature and stirred for an additional twelve hours. The reaction mixture was added to 10 liters of ice and acidified to pH 4 with conc. hydrochloric acid. A solid which precipitated was collected by filtration, and the filtrate was extracted twice with 1000 ml. portions of dichloromethane. The extracts were combined and added to the solid precipitate which had been collected, and the organic solution was then washed with water and then with brine, dried, and concentrated to dryness under reduced pressure to provide 926 g. (89% yield) of 5-*tert*.-butyl-3-(N-phenoxycarbonylamino)isoxazole.

B. A solution of 500 g. of 5-*tert*.-butyl-3-(N-phenoxycarbonylamino)isoxazole in 9.25 liters of toluene containing 500 g. of N-methyl-(2,2-dimethoxy)-ethylamine was heated at reflux (108-110°C.) for three

hours. The reaction mixture was cooled to room temperature, and the solvent was removed by evaporation under reduced pressure to give an oil. The oil was dissolved in 4 liters of ethyl acetate, ahd the organic solution was washed six times with 1 liter portions of 1N sodium hydroxide. The organic solvent was then removed by evaporation, and the oil which remained was crystallized from 2 liters of hexane. The solid was collected by filtration, washed with fresh hexane and air dried to give 359 g. (65.6% yield) of 1-(5-tert.-butyl-3-isoxazolyl)-3-[2,2-(dimethoxy)ethyl]-3-methylurea.

C. A solution of 359 g. of the urea thus prepared in 2400 ml. of 0.5N hydrochloric acid was heated at 50-54°C. for fifty minutes. The reaction mixture was then added to 8 liters of ice. A precipitate formed which was collected by filtration and then dissolved in 11 liters of ethyl acetate, washed with water and then with brine, dried, and the solvent was removed by evaporation under reduced pressure to give 284 g. (94.3% yield) of 1-(5-tert.-butyl-3-isoxazolyl)-4-hydroxy-1-methyl-2-imidazolidinone.

M.P. 176-178°C.

Analysis calc. for $C_{11}H_{17}N_3O_3$

Theory: C, 55.22; H, 7.16; N, 17.56.

Found: C, 55.52; H, 7.26; N, 17.30.

Example 2

3-(5-methyl-3-isoxazolyl)-4-hydroxy-1-methyl-2-imidazolidinone.

A. To a cold stirred solution of 29.4 g. of 3-amino-5-methylisoxazole in 500 ml. of pyridine were added dropwise over one hour 55 g. of phenyl chloroformate. The reaction mixture was stirred for one hour at 0°C. following the addition, and then for sixteen hours at room temperature. The reaction mixture next was diluted with 200 ml. of water, and made acidic to pH 2 by the addition of concentrated hydrochloric acid. The white precipitate which formed was collected by filtration and dried to give 37 g. of 3-(N-phenoxycarbonylamino)-5-methylisoxazole.

M.P. 158-160°C.

B. Preparation of 1-(5-methyl-3-isoxazolyl)-3-(2,2-dimethoxyethyl)-3-methylurea.

A solution of 70 g. of 3-(N-phenoxycarbonylamino)-5-methylisoxazole in 750 ml. of toluene containing 45 g. of N-methyl-2,2-dimethoxyethylamine was heated at reflux for three hours. After cooling the reaction mixture to room-temperature, the solvent was removed by evaporation under reduced pressure to provide a dark oil. The oil was chromatographed over silica gel, eluting with diethyl ether. The fractions shown by thin layer chromatographic analysis to contain the desired product were combined, and the solvent was removed by evaporation under reduced pressure to give 29 g. of 1-(5-methyl-3-isoxazolyl)-3-(2,2-dimethoxyethyl)-3-methylurea.

M.P. 89-90°C.

Analysis calculated for $C_{10}H_{17}N_3O_4$

Theory: C, 49.37; H, 7.04; N, 17.27.

Found: C, 49.10; H, 6.86; N, 17.02.

C. 3-(5-methyl-3-isoxazolyl)-4-hydroxy-1-methyl-2-imidazolidinone.

A solution of 10 g. of 1-(5-methyl-3-isoxazolyl)-3-(2,2-dimethoxyethyl)-3-methylurea in 100 ml. of 1N hydrochloric acid was heated at 50°C. for forty-five minutes. The hot solution was added to 100 g. of ice, and the precipitate which formed was collected by filtration and identified as 2.8 g. of 3-(5-methyl-3-isoxazolyl)-4-hydroxy-1-methyl-2-imidazolidinone.

M.P. 144-145°C.

Analysis calculated for $C_8H_{11}N_3O_3$

Theory: C, 48.73; H, 5.62; N, 21.31.

Found: C, 48.92; H, 5.33; N, 21.58.

Example 3

3-(5-isopropyl-3-isoxazolyl)-4-hydroxy-1-methyl-2-imidazolidinone.

A. Preparation of 5-isopropyl-3-(N-phenoxycarbonylamino)isoxazole.

To a cold stirred solution of 8 g. of 3-amino-5-isopropylisoxazole in 100 ml. of pyridine were added dropwise over thirty minutes 11 g. of phenyl chloroformate. Following complete addition, the reaction mixture was stirred at 0°C. for one hour, and

then was warmed to room temperature and stirred for an additional five hours. The reaction mixture was next added to 50 g. of ice and made acidic by the addition of concentrated hydrochloric acid. The solid precipitate was collected by filtration to provide 14.2 g. of 5-isopropyl-3-(N-phenoxycarbonylamino)isoxazole.

M.P. 109-113°C.

B. Preparation of 1-(5-isopropyl-3-isoxazolyl)-3-(2,2-dimethoxyethyl)-3-methylurea.

A solution of 8 g. of N-methyl-2,2-dimethoxyethylamine in 150 ml. of toluene containing 14.2 g. of 5-isopropyl-3-(N-phenoxycarbonylamino)-isoxazole was heated at reflux for three hours. After cooling to room temperature, the reaction mixture was concentrated in volume by evaporation of the solvent under reduced pressure to provide an oil. The oil was purified by chromatography over silica gel, eluting with diethyl ether. The appropriate fractions were collected and the solvent was evaporated therefrom to provide 9.0 g. of 1-(5-isopropyl-3-isoxazolyl)-3-(2,2-dimethoxyethyl)-3-methylurea.

M.P. 68-71°C.

C. A solution of 5 g. of 1-(5-isopropyl-3-isoxazolyl)-3-(2,2-dimethoxyethyl)-3-methylurea in 75 ml. of 1N hydrochloric acid was heated at 40°C. for forty-five minutes. The reaction mixture then was added to 50 g. of ice water, and the precipitated product was collected by filtration and dried to give 2.4 g. of 3-(5-isopropyl-3-isoxazolyl)-4-hydroxy-1-methyl-2-imidazolidinone.

M.P. 145-146°C.

Analysis calculated for $C_{10}H_{15}N_3O_3$

Theory: C, 53.32; H, 6.71; N, 18.66.

Found: C, 53.11; H, 6.51; N, 18.43.

## Example 4

3-(3-*tert*.-butyl-5-isoxazolyl)-4-hydroxy-1-methyl-2-imidazolidinone

A. Preparation of 3-*tert*.-butyl-5-(N-phenoxycarbonylamino)-isoxazole.

A solution of 16.0 g. of 4,4-dimethyl-3-oxovaleronitrile in 500 ml. of ethanol containing 50.0 g. of sodium acetate and 38.0 g. of hydroxylamine hydrochloride dissolved in 500 ml. of water was heated at reflux for 2 hours. The aqueous mixture was cooled and concentrated to a volume of about 500 ml. A solid crystallized and was collected by filtration and dried to give 14.1 g. of 5-amino-3-*tert*.-butylisoxazole. M.P. 85-88°C.

To a cold (0°C.) stirred solution of 14.1 g. of 5-amino-3-*tert*.-butylisoxazole in 250 ml. of pyridine were added dropwise over thirty minutes 17.5 g. of phenyl chloroformate. The reaction mixture next was stirred at 0°C. for two hours, and then warmed to room temperature and stirred for an additional twelve hours. The reaction mixture was next added to 500 g. of ice and neutralized with concentrated hydrochloric acid. The aqueous mixture was extracted several times with ethyl acetate. The extracts were combined, dried, and the solvent was removed by evaporation under reduced pressure to afford 14.2 g. of 5-phenoxycarbonylamino-3-*tert*.-butylisoxazole.

B. Preparation of 1-(3-*tert*.-butyl-5-isoxazolyl)-3-methyl-3-(2,2-dimethoxyethyl)urea.

A solution of 14.2 g. of 5-phenoxycarbonylamino-3-*tert*.-butylisoxazole and 15.0 g. of N-methyl-2,2-dimethoxyethylamine in 250 ml. of toluene was heated at reflux for three hours. The reaction mixture was cooled to room temperature and concentrated to an oil by evaporation of the solvent under reduced pressure. The oil was purified twice by chromatography over a silica gel column, eluting with diethyl ether, to give 4.0 g. of 1-(3-*tert*.-butyl-5-isoxazolyl)-3-methyl-3-(2,2-dimethoxyethyl)urea (about 95% pure).

Analysis calculated for $C_{13}H_{23}N_3O_4$

Theory: C, 54.72; H, 8.12; N, 14.73.

Found: C, 54.50; H, 7.85; N, 14.48.

C. 3-(3-*tert*.-butyl-5-isoxazolyl)-4-hydroxy-1-methyl-2-imidazolidinone.

A solution of 3.2 g. of 1-(3-*tert*.-butyl-5-isoxazolyl)-3-methyl-3-(2,2-dimethoxyethyl)urea in 20 ml. of 0.5N hydrochloric acid was heated at 50°C. and stirred for thirty minutes. The acidic mixture was then added to 50 g. of ice. The aqueous mixture was filtered, and the solid precipitate was crystallized from ethyl acetate to give 1.2 g. of 3-(3-*tert*.-butyl-5-isoxazolyl)-4-hydroxy-1-methyl-2-imidazolidinone.

M.P. 128-130°C.

Analysis calculated for $C_{11}H_{17}N_3O_3$

Theory: C, 55.22; H, 7.16; N, 17.56.

Found: C, 54.91; H, 6.91; N, 17.34.

Example 5

3-(3-methyl-5-isoxazolyl)-4-hydroxy-1-methyl-2-imidazolidinone

A. 5-Phenoxycarbonylamino-3-methylisoxazole was prepared by reacting 5-amino-3-methylisoxazole with phenyl chloroformate in pyridine. To a solution of 38.0 g. of 5-phenoxycarbonylamino-3-methylisoxazole in 500 ml. of toluene were added in one portion 20.0 g. of N-methyl-2,2-dimethoxyethylamine. The reaction mixture was heated at reflux for four hours, and then was cooled to room temperature and concentrated to an oil. Purification of the oil by chromatography over silica gel, followed by crystallization from ethyl acetate, afforded 12.1 g. of 1-(3-methyl-5-isoxazolyl)-3-methyl-3-(2,2-dimethoxyethyl)urea.

M.P. 68-70°C.

Analysis calculated for $C_{10}H_{17}N_3O_4$

Theory: C, 49.37; H, 7.04; N, 17.27.

Found: C, 49.22; H, 6.86; N, 17.44.

B. A solution of 6.0 g. of 1-(3-methyl-5-isoxazolyl)-3-methyl-3-(2,2-dimethoxyethyl)urea in 100 ml. of 1N hydrochloric acid was heated at 40°C. for forty-five minutes. The reaction mixture was cooled, and the precipitate which formed was collected by filtration and air dried to give 4.0 g. of 3-(3-methyl-5-isoxazolyl)-4-hydroxy-1-methyl-2-imidazolidinone.

M.P. 184-186°C.

Analysis calculated for $C_8H_{11}N_3O_3$

Theory: C, 48.73; H, 5.62; N, 21.31.

Found: C, 48.96; H, 5.58; N, 21.18.

Example 6

3-(3-isopropyl-5-isoxazolyl)-4-hydroxy-1-methyl-2-imidazolidinone

A solution of 12.4 g. of 5-phenoxycarbonylamino-3-isopropylisoxazole and 15.0 g. of N-methyl-2,2-dimethoxyethylamine in 200 ml. of toluene was heated at reflux for three hours. The reaction mixture then was cooled and the solvent was removed by evaporation under reduced pressure to provide an oil. Purification of the oil by chromatography over silica gel afforded 3.2 g. of 1-(3-isopropyl-5-isoxazolyl)-3-methyl-3-(2,2-dimethoxyethyl)urea.

Two grams of the urea thus prepared were dissolved in 25 ml. of 1N hydrochloric acid, and the mixture was heated at 40°C. and stirred for thirty minutes. The acidic reaction mixture was added to 50 g. of ice, and the resulting aqueous solution was extracted with ethyl acetate. The extract was dried and the solvent was removed by evaporation under reduced pressure to give 1.3 g. of the product as a brown solid. The solid was recrystallized from fresh ethyl acetate to give, as a white crystalline solid, 3-(3-isopropyl-5-isoxazolyl)-4-hydroxy-1-methyl-2-imidazolidinone.

M.P. 152-154°C.

Analysis calculated for $C_{10}H_{15}N_3O_3$

Theory: C, 53.32; H, 6.71; N, 18.66.

Found: C, 53.32; H, 6.59; N, 18.85.

Example 7

3-[3-(1,1-dimethyl-2-methoxyethyl)-5-isoxazolyl]-4-hydroxy-1-methyl-2-imidazolidinone

A solution of 6.0 g. of 5-phenoxycarbonylamino-3-(1,1-dimethyl-2-methoxyethyl)isoxazole in 100 ml. of toluene containing 3.0 g. of N-methyl-2,2-dimethoxyethylamine was heated at reflux for two hours. After cooling the reaction mixture to room temperature and removing the solvent by evaporation under reduced pressure, the oil that was formed was chromatographed over silica gel to provide 4.3 g. of 1-[3-(1,1-dimethyl-2-methoxyethyl)-5-isoxazolyl]-3-methyl-3-(2,2-dimethoxyethyl)urea.

A solution of 4.3 g. of the urea thus formed in 50 ml. of 1N hydrochloric acid was heated to 40°C. and stirred for one and one-half hours. The acidic reaction mixture was then added to 50 g. of ice, and the aqueous acid solution was extracted with ethyl acetate. The ethyl acetate layer was separated and dried, and the solvent was removed by evaporation under reduced pressure to give an oil. The oil was further purified by chromatography over silica gel to provide, after crystallization from fresh ethyl acetate, 0.4 g. of 3-[3-(1,1-dimethyl-2-methoxyethyl)-5-isoxazolyl]-4-hydroxy-1-methyl-2-imidazolidinone.

M.P. 87-88°C.

Analysis calculated for $C_{12}H_{19}N_3O_4$

Theory: C, 53.52; H, 7.11; N, 15.60.

Found: C, 53.64; H, 6.92; N, 15.45.

Example 8

3-(3-methyl-4-cyano-5-isoxazolyl)-4-hydroxy-1-methyl-2-imidazolidinone

A. Preparation of 5-phenoxycarbonylamino-3-methyl-4-cyanoisoxazole.

To a cold (0°C.) stirred solution of 28.0 g. of 5-amino-3-methyl-4-cyanoisoxazole in 500 ml. of pyridine were added dropwise over thirty minutes 50.0 g. of phenyl chloroformate. Following the addition, the reaction mixture was warmed to room temperature and stirred for fourteen hours. The mixture was added to 500 g. of ice, and the solid which precipitated was collected, air dried, and identified as 27.0 g. of 5-phenoxycarbonylamino-3-methyl-4-cyanoisoxazole.

M.P. 96-118°C.

B. A solution of 40.0 g. of N-methyl-2,2-dimethoxyethylamine in 500 ml. of toluene containing 40.0 g. of 5-phenoxycarbonylamino-3-methyl-4-cyanoisoxazole, prepared as described above, was heated at reflux for three hours. The reaction mixture was cooled to room temperature and concentrated to an oil. The oil was chromatographed over silica gel, eluting with diethyl ether. The appropriate fractions were collected, combined, and the solvent was removed therefrom by evaporation under reduced pressure to give 22.0 g. of the product as a solid. The solid was slurried with fresh diethyl ether to give 1-(3-methyl-4-cyano-5-isoxazolyl)-3-methyl-3-(2,2-dimethoxyethyl)-urea.

M.P. 126-127°C.

Analysis calculated for $C_{11}H_{16}N_4O_4$

Theory: C, 49.25; H, 6.01; N, 20.88.

Found: C, 49.34; H, 5.82; N, 20.65.

C. A solution of 4.0 g. of 1-(3-methyl-4-cyano-5-isoxazolyl)-3-methyl-3-(2,2-dimethoxyethyl)-urea in 40 ml. of 1N hydrochloric acid was heated at 40°C. and stirred for two hours. The reaction mixture was then added to 100 ml. of ice water, and the aqueous mixture was extracted with ethyl acetate. The organic layer was separated, dried, and the solvent was removed by evaporation under reduced pressure to provide a yellow solid. Recrystallization of the solid from fresh ethyl acetate gave 2.4 g. of 3-(3-methyl-4-cyano-5-isoxazolyl)-4-hydroxy-1-methyl-2-imidazolidinone.

M.P. 120-121°C.

Analysis calculated for $C_9H_{10}N_4O_3$

Theory: C, 48.65; H, 4.54; N, 25.21.

Found: C, 48.76; H, 4.37; N, 25.12.

## Example 9

3-(3-tert.-butyl-5-isoxazolyl)-4-acetoxy-1-methyl-2-imidazolidinone

To a cold (0°C.) stirred solution of 900 mg. of 3-(3-tert.-butyl-5-isoxazolyl)-4-hydroxy-1-methyl-2-imidazolidinone (prepared according to Example 4) in 20 ml. of dichloromethane containing 400 mg. of pyridine was added dropwise over twenty minutes a solution of 400 mg. of acetyl chloride in 20 ml. of dichloromethane.

The reaction mixture was stirred for two hours at 0°C. following the addition, and then the solvent was removed by evaporation under reduced pressure. The solid thus formed was washed with water and then crystallized from ethanol to give 900 mg. of 3-(3-*tert*.-butyl-5-isoxazolyl)-4-acetoxy-1-methyl-2-imidazolidinone.

M.P. 105-107°C.

Analysis calculated for $C_{13}H_{19}N_3O_4$

Theory: C, 55.51; H, 6.81; N, 14.94.

Found: C, 55.79; H, 6.56; N, 15.24.

## Example 10

Following the general procedure of Example 9, 2.5 g. of 3-(5-*tert*.-butyl-3-isoxazolyl)-4-hydroxy-1-methyl-2-imidazolidinone from Example 1 was reacted with 1.1 g. of acetyl chloride and 1.1 g. of pyridine to give 1.7 g. of 3-(5-*tert*.-butyl-3-isoxazolyl)-4-acetoxy-1-methyl-2-imidazolidinone.

M.P. 113-116°C.

Analysis calculated for $C_{13}H_{19}N_3O_4$

Theory: C, 55.51; H, 6.81; N, 14.94.

Found: C, 55.25; H, 6.78; N, 14.73.

## Example 11

3-(3,4-dimethyl-5-isoxazolyl)-1-methyl-2-imidazolinone

3,4-Dimethyl-5-aminoisoxazole was reacted with phenyl chloroformate and pyridine to give 3,4-dimethyl-5-(N-phenoxycarbonylamino)isoxazole, which

when reacted with N-methyl-2,2-dimethoxyethylamine afforded 1-(3,4-dimethyl-5-isoxazolyl)-3-(2,2-dimethoxyethyl)-3-methylurea.

A solution of 5.0 g. of 1-(3,4-dimethyl-5-isoxazolyl)-3-(2,2-dimethoxyethyl)-3-methylurea in 50 ml. of 2N hydrochloric acid was heated at 40°C. for two hours. The reaction mixture then was added to 50 g. of ice, and the aqueous mixture was extracted several times with ethyl acetate. The organic extracts were combined, washed with water, dried, and the solvent was removed by evaporation under reduced pressure to provide 3.8 g. of a white solid. The solid was recrystallized from fresh ethyl acetate to afford 2.7 g. of 3-(3,4-dimethyl-5-isoxazolyl)-1-methyl-2-imidazolinone.

M.P. 89-91°C.

Analysis calculated for $C_9H_{11}N_3O_2$

Theory: C, 55.95; H, 5.74; N, 21.75

Found: C, 55.76; H, 5.53; N, 21.60.

Example 12

3-[5-(1-ethyl-1-methylpropyl)-3-isoxazolyl]-4-hydroxy-1-methyl-2-imidazolidinone

5-(1-Ethyl-1-methylpropyl)-3-aminoisoxazole (8.41 g.) was reacted with a 0.1 molar excess of phenyl chloroformate (8.61 g.) by the general procedure of Example 1 (A) to give 16.35 g. of 5-(1-ethyl-1-methylpropyl)-3-(N-phenoxycarbonylamino)isoxazole (88% yield). Fourteen and one-half grams of the isoxazole carbamate thus formed was reacted with 2.3 molar excess (17.67 g.)

of N-methyl-(2,2-diethoxy)ethylamine according to the procedure of Example 1 (B) to give 1-[5-(1-ethyl-1-methylpropyl)-3-isoxazolyl]-3-[2,2-(diethoxy)ethyl]-3-methylurea.

Reaction of 0.5N hydrochloric acid with the isoxazolylurea thus formed according to the general method of Example 1 (C) provided, after crystallization from diethyl ether and chromatography over 200 g. of silica gel, 742 mg. of 3-[5-(1-ethyl-1-methylpropyl)-3-isoxazolyl]-4-hydroxy-1-methyl-2-imidazolidinone.

M.P. 98-99°C.

Analysis calculated for $C_{13}H_{21}N_3O_3$

Theory: C, 58.41; H, 7.92; N, 15.72.

Found: C, 58.11; H, 7.86; N, 15.46.

## Example 13

3-[5-(1-ethyl-1-methylpropyl)-3-isoxazolyl]-4-acetoxy-1-methyl-2-imidazolidinone

Acetyl chloride (2.2 g.) was reacted with 5.35 g. of 3-[5-1-ethyl-1-methylpropyl)-3-isoxazolyl]-4-hydroxy-1-methyl-2-imidazolidinone prepared according to the method of Example 12 by following the general procedure of Example 9 to provide, following crystallization from Skelly B and diethyl ether, 4.86 g. of 3-[5-(1-ethyl-1-methylpropyl)-3-isoxazolyl]-4-acetoxy-1-methyl-2-imidazolidinone. (78.5% yield).

M.P. 70-72°C.

Analysis calculated for $C_{15}H_{23}N_3O_4$

Theory: C, 54.24; H, 7.49; N, 13.58.

Found: C, 54.06; H, 7.28; N, 13.39.

Example 14

3-[5-(1-ethylcyclohexyl)-3-isoxazolyl]-4-hydroxy-1-methyl-2-imidazolidinone

A solution of 19.43 g. of 3-amino-5-(1-ethylcyclohexyl)isoxazole in 100 ml. of toluene was cooled to 5°C. and stirred while hydrogen chloride was bubbled through it for fifteen minutes. A solution of 29.68 g. of phosgene in 125 ml. of cold toluene was then added to the reaction mixture dropwise over thirty minutes. Following complete addition, the reaction mixture was heated to 60°C. and stirred at that temperature for six hours, and then heated to 80°C. and stirred for one additional hour. The reaction mixture next was cooled to room temperature and the solvent was removed by evaporation under reduced pressure to provide [5-(1-ethylcyclohexyl)-3-isoxazolyl]isocyanate as an orange oil.

The oil thus formed was dissolved in 250 ml. of dry toluene and stirred at room temperature while 44.16 g. of N-methyl-2,2-dimethoxyethylamine were added slowly over ten minutes. The reaction mixture was then heated to 55°C. and stirred for sixteen hours. After cooling the mixture to room temperature, it was washed with dilute aqueous hydrochloric acid, with brine, and then dried. Removal of the solvent by evaporation under reduced pressure gave 39 g. of 1-[5-(1-ethylcyclohexyl)-3-isoxazolyl]-3-(2,2-dimethoxyethyl)-3-methylurea as a red oil.

A solution comprised of 36.75 g. of the urea thus formed, 200 ml. of 0.5 N hydrochloric acid and 400

ml. of diethyl ether was heated at 55°C. for sixteen hours, allowing the diethyl ether to evaporate. The reaction mixture was then dissolved in fresh diethyl ether, treated with decolorizing carbon, and the excess solvent was removed by evaporation under reduced pressure to provide an oil. The oil was crystallized from diethyl ether and Skelly B to give 16 g. of 3-[5-(1-ethylcyclohexyl)-3-isoxazolyl]-4-hydroxy-1-methyl-2-imidazolidinone (53% yield from the urea intermediate). M.P. 93-95°C.

Analysis calculated for $C_{15}H_{23}N_3O_3$

Theory: C, 61.41; H, 7.90; N, 14.32.

Found: C, 61.68; H, 8.09; N, 14.06.

Example 15

3-[5-(1-ethylcyclohexyl)-3-isoxazolyl]-4-acetoxy-1-methyl-2-imidazolidinone

Acetyl chloride (2.2 g.) was reacted with 6.05 g. of 3-[5-(1-ethylcyclohexyl)-3-isoxazolyl]-4-hydroxy-1-methyl-2-imidazolidinone from Example 14 in pyridine and dichloromethane to give, following crystallization from Skelly B and diethyl ether, 4.12 g. of 3-[5-(1-ethylcyclohexyl)-3-isoxazolyl]-4-acetoxy-1-methyl-2-imidazolidinone. (57% yield).

M.P. 95-97°C.

Analysis calculated for $C_{17}H_{25}N_3O_4$

Theory: C, 60.88; H, 7.51; N, 12.53.

Found: C, 60.61; H, 7.47; N, 12.70.

Example 16

3-(5-n-propyl-3-isoxazolyl)-4-hydroxy-1-methyl-2-imidazolidinone

Following the general procedure of Example 11, 7.89 g. of 3-amino-5-n-propylisoxazole were reacted with 17.81 g. of phosgene and hydrogen chloride to provide, in situ, (5-n-propyl-3-isoxazolyl)isocyanate. The isocyanate, without isolation, was then reacted with 26.5 g. of N-methyl-2,2-dimethoxyethylamine to give 18.0 g. of 1-(5-n-propyl-3-isoxazolyl)-3-(2,2-dimethoxyethyl)-3-methylurea as a reddish-orange oil.

To a solution of 18.0 g. of the urea thus formed in 50 ml. of diethyl ether was added in one portion 100 ml. of 1N hydrochloric acid. The reaction mixture was heated for fifteen minutes at 50-55°C., under a nitrogen atmosphere to aid removal of diethyl ether by evaporation. The reaction mixture was then diluted with cold water, and the precipitate which had formed was collected by filtration, washed with fresh water, and then dissolved in ethyl acetate and dried. The solvent was removed by evaporation to give a solid, and the solid then was crystallized twice from dichloromethane and diethyl ether to afford 6.18 g. of 3-(5-n-propyl-3-isoxazolyl)-4-hydroxy-1-methyl-2-imidazolidinone.

M.P. 122-124°C.

Analysis calculated for $C_{10}H_{17}N_3O_3$

Theory: C, 52.85; H, 7.54; N, 18.49.

Found: C, 52.95; H, 7.35; N, 18.37.

Example 17

3-(5-n-propyl-3-isoxazolyl)-4-acetoxy-1-methyl-2-imidazolidinone

Following the general procedure of Example 9, 3.41 g. of 3-(5-n-propyl-3-isoxazolyl)-4-hydroxy-1-methyl-2-imidazolidinone from Example 16 were reacted with 1.6 g. of acetyl chloride and 2.0 g. of pyridine in dichloromethane to give 3.5 g. of 3-(5-n-propyl-3-isoxazolyl)-4-acetoxy-1-methyl-2-imidazolidinone. (86.6% yield).

M.P. 70-72°C.

Analysis calculated for $C_{12}H_{17}N_3O_4$

Theory: C, 53.92; H, 6.41; N, 15.72.

Found: C, 53.65; H, 6.13; N, 15.73.

The isoxazolylimidazolidinone derivatives provided by this invention exhibit both terrestrial and aquatic herbicidal and plant growth regulator activity, and accordingly are useful in the control and elimination of unwanted vegetative growth. The compounds are nontoxic to a number of beneficial terrestrial plants, including cotton, corn, soybean, wheat, rice, barley, oats, peanuts and related crops raised for both human and domestic animal consumption.
The herbicides of the invention are effective terrestrially in both pre-emergent and early post-emergent control of a wide variety of grasses and broadleaf weeds. Commonly encountered unwanted terrestrial vegetation which is subject to control with the herbicidal isoxazolylimidazolidinones and isoxazolylimidazolinones of this invention include annuals such

as pigweed, lambsquarter, wild oats, velvetleaf, ryegrass, goose grass, crabgrass, foxtail, chickweed, barnyardgrass, smartweed, knotweed, cocklebur, kochia, medic, ragweed, hemp nettle, spurge, jungle rice, morningglory, ducksalad, cheatgrass, fall panicum, jimsonweed, watergrass, spangletop, and the like. Biennials subject to control include wild carrot, wild barley, burdock, bull thistle, houndstongue, and purple star thistle. Other common weeds which can be controlled with the herbicides of this invention include perennial ryegrass, quackgrass, Johnson grass, sheep sorrell, nutgrass, field chickweed, dandelion, Russian Knotweed, horsetail, sesbania, cattail, wintercress, nutsedge, milkweed, sicklepod, and related weeds.

The pre-emergent and post-emergent terrestrial herbicidal activity of several isoxazolylimidazolidinone derivatives of this invention has been determined in standard greenhouse experiments. Such experiments were carried out by first formulating a test compound for convenient soil surface or overtop foliar spray application. The test compound was formulated by dissolving 0.2 percent by weight in a solution containing 4.0 percent by weight acetone, 4.0 percent by weight ethanol, and 91.7 percent by weight of deionized water, and 0.1 percent by weight of a commercial surfactant, for example Toximul R and S, which are blends of anionic and nonionic surface active agents (Stepan Chemical Co. Northfield, Illinois, 60093). The solution containing the test compound was then serially diluted with deionized water to the appropriate volume containing the desired concentration of test compound. The formulated compounds were applied pre-emergence and/or post-emergence to metal greenhouse flats filled with soil and seeded to test plants. The compounds were evaluated at several application rates, initially at 15 pounds per acre (16.8 kg/ha) and at lower rates until compound activity diminished. Pre-emergence applications were made to the soil surface of the seeded flats. Post-emergence applications were made to the foliage of the various plant species approximately twelve days after seeding. All treated flats were maintained in a greenhouse following treatment. Evaluations of compound activity were made in the form of plant injury rating on a scale of 1 to 5. The rating of "1" refers to no plant injury; "2" slight injury; "3" moderate injury; "4" severe injury; and "5" death to all treated plants. Evaluations were made from 11 to 14 days after post-emergence application, and from 18 to 21 days following pre-emergence application.

Tables 1-10 which follow presents the herbicidal activity of compounds provided by this invention.

Table 1

Plant Injury Ratings for 3-[5-(1,1-dimethylethyl)-3-isoxazolyl]-4-hydroxy-1-methyl-2-imidazolidinone from Example 1

PLANT SPECIES

| | | Pre-emergence | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 4 | 5 | 4 | 4 | 5 | 5 | 4 | 5 | 4 | 5 | - | 5 | 4 | 5 | 5 | 5 | 5 | 4 | 5 | 5 | 5 | 5 | 5 | 5 | 5 | 5 | 5 |
| 2 | (2.7) | 4 | 4 | 4 | 4 | 5 | 5 | 4 | 5 | 4 | 4 | - | 5 | 4 | 5 | 5 | 5 | 4 | 4 | 5 | 5 | 5 | 5 | 5 | 5 | 5 | 5 | 5 |
| 1 | (1.12) | 4 | 4 | 3 | 3 | 5 | 5 | 4 | 5 | 4 | 4 | - | 4 | 4 | 5 | 4 | 4 | 4 | 4 | 5 | 5 | 4 | 4 | 4 | 5 | 4 | 5 | 5 |
| 0.5 | (0.56) | 1 | 2 | 2 | 3 | 5 | 5 | 3 | 5 | 3 | 3 | 5 | 3 | 4 | 5 | 5 | 5 | 5 | 2 | 3 | 5 | 3 | 4 | 4 | 3 | 5 | 4 | 5 |
| 0.25 | (0.28) | 1 | 1 | 1 | 1 | 3 | 5 | 2 | 3 | 3 | 3 | 5 | 4 | 4 | 5 | 4 | 2 | 4 | 2 | 2 | 4 | 1 | 3 | 3 | 3 | 3 | 3 | 4 |
| 0.125 | (0.14) | 1 | 1 | 1 | 1 | 1 | 4 | 2 | 2 | 2 | 1 | - | 2 | 2 | 3 | 3 | 2 | 5 | 2 | 1 | 5 | | | | | | | |
| 0.0625 | (0.07) | 1 | 1 | 1 | 1 | 1 | 1 | 1 | 2 | 1 | 1 | - | 3 | 1 | 3 | 3 | 1 | 1 | 1 | 1 | 1 | | | | | | | |

Table 2

Plant Injury Ratings for 3-(5-methyl-3-isoxazolyl)-
4-hydroxy-1-methyl-2-imidazolidinone of Example 2

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 1 | 1 | 2 | 1 | 1 | 4 | 1 | 2 | 2 | 2 | 4 | 2 | 1 | 3 | 2 | 1 | 4 | 1 | 1 | 2 | 2 | 2 | 3 | 2 | 3 | 3 | 2 |
| 2 | (2.7) | 1 | 1 | 1 | 1 | 1 | 2 | 1 | 2 | 1 | 1 | 4 | 3 | 1 | 2 | 2 | 1 | 4 | 1 | 1 | 1 | 2 | 2 | 3 | 2 | 3 | 2 | 2 |
| 1 | (1.12) | 1 | 1 | 1 | 1 | 1 | 1 | 1 | 2 | 1 | 1 | 2 | 2 | 1 | 1 | 2 | 1 | 3 | 1 | 1 | 1 | 1 | 2 | 3 | 1 | 3 | 2 | 2 |

## Table 3

Plant Injury Ratings for 3-(5-isopropyl-3-isoxazolyl)-4-hydroxy-1-methyl-2-imidazolidinone of Example 3

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 4 | (4.5) | 3 | 3 | 3 | 4 | 4 | 4 | 3 | 5 | 4 | 3 | 5 | 4 | 3 | 4 | 5 | 5 | 5 | 3 | 5 | 5 |
| 2 | (2.7) | 2 | 2 | 4 | 5 | 4 | 4 | 2 | 5 | 4 | 4 | 5 | 4 | 3 | 5 | 5 | 4 | 5 | 3 | 4 | 5 |
| 1 | (1.12) | 1 | 1 | 2 | 2 | 4 | 4 | 1 | 5 | 3 | 2 | 5 | 4 | 3 | 4 | 5 | 3 | 5 | 2 | 5 | 5 |
| 0.5 | (0.56) | 2 | 2 | 3 | 3 | 4 | 4 | 3 | 5 | 4 | 3 | 5 | 4 | 4 | 4 | 4 | 4 | 5 | 2 | 3 | 4 |
| 0.25 | (0.28) | 1 | 1 | 1 | 1 | 2 | 2 | 2 | 3 | 2 | 1 | 5 | 3 | 4 | 4 | 4 | 3 | 5 | 3 | 3 | 4 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 4 | (4.5) | 5 | 4 | 5 | 4 | 5 | 4 | 5 |
| 2 | (2.7) | 5 | 4 | 5 | 4 | 5 | 4 | 5 |
| 1 | (1.12) | 3 | 4 | 5 | 4 | 5 | 4 | 4 |
| 0.5 | (0.56) | 4 | 5 | 5 | 3 | 5 | 4 | 4 |
| 0.25 | (0.28) | 2 | 3 | 5 | 2 | 4 | 3 | 3 |

## Table 4

Plant Injury Ratings for 3-(3-tert.-butyl-5-isoxazolyl)-4-hydroxy-1-methyl-2-imidazolidinone of Example 4

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 4 | (4.5) | 1 | 2 | 3 | 3 | 5 | 4 | 2 | 5 | 3 | 3 | 5 | 4 | 3 | 4 | 4 | 5 | 5 | 3 | 5 | 5 |
| 2 | (2.7) | 1 | 2 | - | 3 | 5 | 5 | 2 | 4 | 3 | 3 | 5 | 3 | 2 | 5 | 4 | 4 | 5 | - | 3 | 5 |
| 1 | (1.12) | 1 | 1 | 3 | 2 | 5 | 4 | 3 | 3 | 3 | 4 | 5 | 4 | 4 | 5 | 3 | 3 | 5 | 5 | 4 | 5 |
| 0.5 | (0.56) | 1 | 1 | 1 | 2 | 4 | 4 | 3 | 2 | 2 | 3 | 5 | 5 | 4 | 5 | 3 | 3 | 5 | 4 | 2 | 4 |
| 0.25 | (0.28) | 1 | 1 | 1 | 1 | 1 | 3 | 2 | 2 | 3 | 1 | 5 | 3 | 3 | 5 | 1 | 2 | 5 | 4 | 2 | 2 |
| 0.125 | (0.14) | 1 | 1 | 1 | 1 | 1 | 2 | 1 | 2 | 1 | 1 | 1 | 1 | 1 | 1 | 3 | 1 | 3 | 1 | 1 | 3 |
| 0.0625 | (0.07) | 1 | 1 | 1 | 1 | 1 | 1 | 1 | 1 | 1 | 1 | 1 | 1 | 1 | 1 | 1 | 1 | 1 | 1 | 1 | 1 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 4 | (4.5) | 3 | 5 | 5 | 5 | 5 | 4 | 5 |
| 2 | (2.7) | 3 | 4 | 5 | 4 | 5 | 4 | 5 |
| 1 | (1.12) | 2 | 3 | 5 | 4 | 5 | 4 | 5 |
| 0.5 | (0.56) | 1 | 2 | 5 | 3 | 5 | 4 | 4 |
| 0.25 | (0.28) | 1 | 2 | 4 | 3 | 4 | 3 | 4 |
| 0.125 | (0.14) | 1 | 2 | 3 | 2 | 3 | 2 | 2 |
| 0.0625 | (0.07) | 1 | 2 | 2 | 2 | 1 | 2 | 2 |

Table 5

Plant Injury Ratings for 3-(3-methyl-5-isoxazolyl)-4-hydroxy-1-methyl-2-imidazolidinone of Example 5

PLANT SPECIES

| appln. rate lbs/A | (kg/ha) | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence: Tomato | Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|---|
| 15 | (16.8) | - | - | - | - | - | - | - | - | 2 | - | - | 1 | - | 3 | - | - | - | - | - | - | 4 | - | 1 | 3 | - | - | - | - |
| 8 | (8.97) | 1 | - | - | - | - | - | - | - | - | - | - | 1 | - | 1 | 1 | - | 1 | - | 1 | 1 | 1 | 1 | 1 | 1 | 1 | 1 | 1 | 1 |

Table 6

Plant Injury Ratings for 3-(3-isopropyl-5-isoxazolyl)-4-hydroxy-1-methyl-2-imidazolidinone of Example 6

PLANT SPECIES

| appln. rate lbs/A | (kg/ha) | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence: Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|
| 2 | (2.7) | 2 | 3 | 2 | 2 | 3 | 5 | 3 | 5 | 2 | 3 | 5 | 1 | 3 | 4 | 3 | 3 | 5 | 2 | 4 | 5 | 1 | 3 | 4 | 3 | 5 | 3 | 3 |
| 1 | (1.12) | 1 | 3 | 2 | 2 | 3 | 5 | 3 | 5 | 2 | 3 | 5 | 1 | 3 | 4 | 2 | 3 | 5 | 2 | 4 | 3 | 1 | 2 | 4 | 2 | 4 | 3 | 2 |
| 0.5 | (0.56) | 1 | 2 | 1 | 1 | 2 | 5 | 2 | 4 | 2 | 3 | 5 | 1 | 2 | 4 | 1 | 3 | 4 | 2 | 2 | 3 | 1 | 2 | 3 | 2 | 3 | 2 | 2 |
| 0.25 | (0.28) | 1 | 1 | 1 | 1 | 1 | 2 | 2 | 3 | 1 | 1 | 5 | 2 | 3 | 3 | 2 | 1 | 3 | 2 | 2 | 3 | | | | | | | |
| 0.0625 | (0.07) | 1 | 2 | - | 1 | 1 | 1 | 1 | 2 | 1 | 1 | 4 | 2 | 1 | 2 | 1 | 1 | 1 | 1 | 1 | 2 | | | | | | | |

## Table 7

Plant Injury Ratings for 3-[3-(1,1-dimethyl-2-methoxyethyl)-5-isoxazolyl]-4-hydroxy-1-methyl-2-imidazolidinone of Example 7

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 4 | (4.5) | 2 | 4 | 3 | 3 | 3 | 4 | 2 | 4 | 2 | 2 | 5 | 4 | 3 | 5 | 5 | 4 | 4 | 2 | 4 | 5 |
| 2 | (2.7) | 1 | 2 | 3 | 4 | 1 | 4 | 2 | 3 | 1 | 2 | 5 | 4 | 2 | 5 | 4 | 3 | 5 | 2 | 3 | 4 |
| 1 | (1.12) | 1 | 2 | 2 | 3 | 1 | 4 | 2 | 2 | 1 | 1 | 5 | 4 | 1 | 5 | 3 | 1 | 4 | 5 | 2 | 2 |
| 0.5 | (0.56) | 1 | 1 | 2 | 1 | 2 | 3 | 1 | 3 | 2 | 1 | 4 | 1 | 2 | 3 | 1 | 1 | 2 | 1 | 1 | 2 |
| 0.25 | (0.28) | 1 | 2 | 1 | 1 | 1 | 3 | 1 | 4 | 1 | 1 | 3 | 1 | 1 | 2 | 1 | 2 | 3 | 1 | 1 | 3 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 4 | (4.5) | 1 | 4 | 4 | 3 | 5 | 2 | 3 |
| 2 | (2.7) | 1 | 3 | 5 | 2 | 4 | 3 | 3 |
| 1 | (1.12) | 1 | 2 | 3 | 3 | 4 | 3 | 3 |
| 0.5 | (0.56) | | | | | | | |
| 0.25 | (0.28) | | | | | | | |

## Table 8

Plant Injury Ratings for 3-(3-methyl-4-cyano-5-isoxazolyl)-4-hydroxy-1-methyl-2-imidazolidinone of Example 8

PLANT SPECIES

| appln. rate lbs/A | (kg/ha) | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 1 | 1 | 1 | 2 | 2 | 2 | 2 | 3 | 1 | 1 | 3 | 2 | 3 | 1 | 1 | 1 | 2 | 1 | 1 | 1 | 1 | 3 | 4 | 3 | 3 | 3 | 4 |
| 2 | (2.7) | 1 | 1 | 1 | 1 | 1 | 3 | 1 | 2 | 1 | 1 | 2 | 2 | 1 | 1 | 1 | 1 | 2 | 1 | 1 | 1 | 1 | 2 | 4 | 2 | 2 | 2 | 3 |
| 1 | (1.12) | 1 | 1 | 1 | 1 | 1 | 1 | 1 | 1 | 1 | 1 | 2 | 3 | 1 | 1 | 1 | 1 | 1 | 1 | 1 | 1 | 1 | 2 | 3 | 2 | 2 | 2 | 2 |

Table 9

Plant Injury Ratings for 3-(3-*tert*-butyl-5-isoxazolyl)-4-acetoxy-1-methyl-2-imidazolidinone of Example 9

PLANT SPECIES

| | | Pre-emergence | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 2 | (2.7) | 2 | 3 | 2 | 2 | 4 | 4 | 4 | 5 | 4 | 4 | 5 | 4 | 5 | 5 | 4 | 3 | 5 | 4 | 4 | 5 | 4 | 3 | 5 | 3 | 5 | 4 | 5 |
| 1 | (1.12) | 2 | 3 | 3 | 3 | 5 | 5 | 4 | 5 | 3 | 4 | 5 | 3 | 4 | 5 | 3 | 2 | 5 | 3 | 2 | 5 | 3 | 3 | 5 | 3 | 5 | 4 | 4 |
| 0.5 | (0.56) | 1 | 1 | 1 | 1 | 3 | 3 | 3 | 3 | 3 | 3 | 4 | 1 | 3 | 3 | 3 | 1 | 5 | 3 | 1 | 4 | 3 | 3 | 5 | 3 | 5 | 3 | 4 |

Table 10

Plant Injury Ratings for 3-(5-tert-butyl-3-isoxazolyl)-4-acetoxy-1-methyl-2-imidazolidinone of Example 10

PLANT SPECIES

| appln. rate lbs/A | (kg/ha) | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|
| 4 | (4.5) | 4 | 5 | 4 | 4 | 5 | 5 | 4 | 5 | 5 | 4 | 5 | 5 | 5 | 5 | 5 | 5 | 5 | 5 | 5 | 5 | 5 | 5 | 5 | 5 | 5 | 5 | 5 |
| 2 | (2.7) | 4 | 5 | 5 | 5 | 5 | 5 | 4 | 5 | 5 | 5 | 5 | 5 | 5 | 5 | 5 | 5 | 5 | 5 | 5 | 5 | 5 | 5 | 5 | 5 | 5 | 5 | 5 |
| 1 | (1.12) | 4 | 3 | 4 | 4 | 5 | 5 | 4 | 5 | 5 | 4 | 5 | 5 | 5 | 5 | 5 | 4 | 5 | 5 | 5 | 5 | 5 | 5 | 5 | 5 | 5 | 5 | 5 |
| 0.5 | (0.56) | 2 | 2 | 2 | 2 | 5 | 4 | 2 | 5 | 5 | 4 | 4 | 1 | 4 | 4 | 5 | 2 | 4 | 4 | 2 | 5 | 3 | 4 | 5 | 5 | 5 | 4 | 5 |
| 0.25 | (0.28) | 1 | - | 2 | 3 | 4 | 2 | 2 | 4 | 4 | 3 | 4 | 2 | 3 | 4 | 5 | 3 | 4 | 2 | 2 | 4 | 2 | 4 | 4 | 4 | 5 | 3 | 5 |
| 0.0625 | (0.07) | 1 | 1 | 1 | 1 | 1 | 1 | 1 | 1 | 1 | 2 | 3 | 3 | 3 | 2 | 3 | 1 | 1 | 1 | 1 | 2 | - | - | - | - | - | - | - |

## Table 11

Plant Injury ratings for 3-(3,4-dimethyl-5-isoxazolyl)-1-methyl-2-imidazolinone of Example 11

PLANT SPECIES

| appln. rate lbs/A | (kg/ha) | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence Tomato | Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|---|
| 15 | (16.8) | - | - | - | - | - | - | - | - | 1 | - | - | 1 | - | 2 | - | - | - | - | - | - | 3 | - | 1 | 2 | - | - | - | - |
| 8 | (8.96) | 1 | - | - | - | - | - | - | - | - | - | - | 1 | - | 2 | 1 | - | 1 | - | 2 | 2 |  | 1 | 1 | 2 | 1 | 1 | 1 | 2 |

Table 12

Plant Injury ratings for 3-[5-(1-ethyl-1-methylpropyl)-3-isoxazoyl]-4-hydroxy-1-methyl-2-imidazolidinone of Example 12

PLANT SPECIES

| appln. rate lbs/A | (kg/ha) | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 8 | (8.96) | 4 | - | - | - | - | - | - | - | - | - | - | 5 | - | 5 | 5 | - | 5 | - | 4 | 5 | 4 | 5 | 5 | 5 | 5 | 5 | 5 |
| 4 | (4.5) | 4 | 5 | 5 | 4 | 5 | 5 | 5 | 5 | 5 | 4 | 5 | 5 | 5 | 5 | 5 | 5 | 5 | 5 | 5 | 5 | 4 | 5 | 5 | 5 | 5 | 5 | 5 |
| 2 | (2.7) | 4 | 4 | 5 | 4 | 5 | 5 | 4 | 5 | 5 | 4 | 5 | 5 | 5 | 5 | 5 | 3 | 5 | 2 | 4 | 5 | 2 | 5 | 5 | 5 | 5 | 5 | 5 |
| 1 | (1.12) | 3 | 3 | 4 | 3 | 4 | 5 | 4 | 5 | 3 | 4 | 3 | 5 | 5 | 5 | 5 | 2 | 5 | 2 | 1 | 2 | 3 | 5 | 5 | 4 | 5 | 5 | 5 |

The data presented in Table 1 demonstrates that 3-[5-(1,1-dimethylethyl)-3-isoxazolyl]-4-hydroxy-1-methyl-2-imidazolidinone is effective both as a pre-emergence and a post-emergence terrestrial herbicide at rates as low as 0.25 pounds per acre (0.28 kg/ha) against weeds such as velvetleaf, zinnia, wildoat, foxtail, pigweed and lambsquarter, while having no or only slight adverse affect on desirable plants such as corn, cotton and soybean.

The data presented in Table 2 demonstrates that the compound tested, while somewhat less active than that of Table 1, nevertheless is an excellent pre-emergence herbicide at rates as low as 2 pounds per acre (2.7 kg/ha) against commonly occurring terrestrial weeds such as lambsquarter and velvetleaf, while displaying little or no toxic affects to desirable plants such as corn, wheat, rice, alfalfa and the like.

Similarly, the data of Table 4 shows that a 3-(5-isoxazolyl)imidazolidinone is toxic both pre- and post-emergence at rates as low as 0.25 pound per acre (0.28 kg/ha) against weeds such as lambsquarter, crabgrass, and pigweed, while having little or no adverse affect on corn, cotton, soybean, rice, or the like.

The data thus far generated indicates that compounds embraced by this invention are useful as selective terrestrial herbicides against common weed varities which are found in desirable crops such as corn, wheat, soybean, and the like. The compounds can also be used in a chemical fallow program, for example in a fallow wheatland weed control program.

Because of the potent terrestrial herbicidal activity possessed by the isoxazolylimidazolidinone derivatives of this invention, they are useful in the elimination and control of the growth of unwanted terrestrial vegetation. The invention therefore further provides a herbicidal method for controlling the growth of unwanted terrestrial vegetation which comprises applying to the plants or to the locus where vegetative control is desired a herbicidally effective amount of an isoxazolylimidazolidinone derivative of this invention as defined above. The practice of such method can be carried out by applying the herbicidal compounds by any of several art-recognized methods. The compounds can be surface applied to soil prior to planting of crops or emergence of seedlings. If desired, the surface applied herbicide can be incorporated into the soil by conventional means. The compounds can alternatively be applied early post-emergence, for instance within about 2 weeks following seedling emergence.

The amount of herbicidal isoxazolylimidazolidinone derivatives to be employed in the method of this invention is an amount which is effective in controlling the growth of unwanted vegetation. Such herbicidal amount will depend upon a number of factors, including the method of application, formulation, soil texture, soil moisture content, the expected population of unwanted vegetation, degree of incorporation, the extent of growth control desired, and related factors. The rate of application normally will be from about 0.01

to about 10.0 pounds per acre, and preferably from about 0.25 to about 5.0 pounds per acre. These ranges are equivalent, respectively, to from about 0.011 to about 11.2 kilograms per hectare, and from about 0.28 to about 5.6 kilograms per hectare.

The isoxazolylimidazolidinone and isoxazolylimidazolinone herbicides to be employed according to the terrestrial method of this invention can be formulated for convenient application as aqueous sprays, drenches, solid powders, dusts and the like. Herbicidal formulations containing an isoxazolylimidazolidinone derivative as defined herein are provided in a further embodiment of this invention. Such formulations will contain from about 1.0 to about 90 percent by weight of active isoxazolylimidazolidinone derivative, and can be prepared by combining an isoxazolylimidazolidinone derivative with any number of common herbicidal carriers, diluents, adjuvants and the like. The formulations can contain liquid carriers and adjuvants such as organic solvents, including benzene, xylene, toluene, a halotoluene such as ortho-chlorotoluene, n-hexane, n-pentane, kerosene, and similar organic solvents. The formulations can additionally contain art-known emulsifiers, stabilizers, dispersants, suspending agents, spreaders, penetrants, wetting agents and the like.

Typical carriers utilized in the dry formulations of the invention include clay, talc, diatomaceous earth, silica, pyrophyllite and the like. Herbicidal dusts are prepared by grinding and blending such solid carriers with a compound of the invention such as 3-(5-tert.-butyl-3-isoxazolyl)-4-hydroxy-5-ethyl-1-

methyl-2-imidazolidinone. Granular formulations provided herein are prepared by impregnating an isoxazolylimidazolidinone derivative onto or into a granulated carrier such as a vermiculite. Such granules typically have a particle size of about 0.2 to about 2.0 mm.

Preferred formulations of the inventions are in the form of wettable powders. Such formulations employ any number of commercially available wetting agents, surfactants and emulsifying agents such as ethoxylated nonyl phenols, ethoxylated octyl phenols, polyoxyethylene stearyl ethers, blends of such agents, and the like. Such formulations can optionally contain adjuvants such as thickening agents, for instance heteropolysaccharide gums and the like such as xanthan gum, as well as antibacterial agents such as aqueous formaldehyde.

The following detailed examples of formulations illustrate preferred aspects of the invention.

## Example 18

Granule

| Ingredient | Percent by Weight |
|---|---|
| 3-(5-isopropyl-3-isoxazolyl)-4-hydroxy-1-ethyl-2-imidazolidinone | 5.0 |
| clay | 95.0 |
| | 100.0 percent |

The isoxazolylimidazolidinone herbicide is substantially dissolved in acetone or similar solvent, and the organic solution is sprayed onto the clay chips. The mixture is thoroughly blended and dried.

Example 19

Dust

| Ingredient | Percent by Weight |
| --- | --- |
| 3-(4-cyano-5-methyl-3-isoxazolyl)-4-acetoxy-5-n-butyl-1-(1,1-dimethylpropyl)-2-imidazolidinone | 40.0 |
| powdered talc | 60.0 |
| | 100.0 percent |

The ingredients are mixed and blended to uniformity and are ground and air-milled to a homogenous, free-flowing dust. The dust may be applied directly to the locus of weed infestation.

Example 20

Wettable Powder

| Ingredient | Percent by Weight |
| --- | --- |
| 3-(3-tert.-butyl-5-isoxazolyl)-4-hydroxy-1-methyl-2-imidazolidinone | 50.0 |
| Fuller's earth | 47.0 |
| methyl cellulose | 0.5 |
| sodium lauryl sulfate | 2.5 |
| | 100.0 |

The above ingredients are blended to uniformity and dispersed in water to the desired concentration of active ingredient.

The herbicidal isoxazolylimidazolidinone derivatives of this invention can be employed in

combination with other herbicides for the effective control of an even broader spectrum of weeds. Well known herbicides which may be utilized together with the herbicides of this invention include diuron, atrazine, trifluralin, metribuzin, simazine, propazine, paraquat, and the like. Combinations of the compounds of this invention together with herbicides which are especially effective against grasses are particularly preferred. Typical of such combinations is trifluralin and a compound such as 3-(4-tert.-butyl-5-isoxazolyl)-1-ethyl-2-imidazolinone. It is contemplated that such combinations will contain the respective herbicides in approximately equal amounts, and that upon application each respective herbicide will be present at about 0.1 to about 5.0 pounds per acre (0.112 to about 5.6 kg/ha).

The isoxazolylimidazolidinones of this invention also are useful in the control of unwanted vegetation in a chemical fallow program, for instance in the control of lambsquarter, morningglory, velvetleaf, and similar weeds in fallow wheatland. Combinations with herbicides such as atrazine and trifluralin are particularly useful in a fallow program.

The compounds defined herein also are useful in regulating the growth pattern of plant life without causing total elimination. In order to achieve such plant growth regulation, the compounds are employed in amounts which are effective to cause growth regulation but are not herbicidal. The compounds are applied directly to the plants or to the locus where the plants to be regulated are growing. The compounds are preferrably formulated for post-emergence over the top

spray application. Typical manifestations of plant growth regulation employing compounds of this invention include control of flowering, control of fruiting, delay in maturation, reduction in stem growth, improvements in yield, and related regulatory responses.

The plant growth regulating properties of the compounds defined herein have been determined by the following test procedure. Test compounds were formulated as a foliar spray by dissolving 50 mg. in 25 ml. of Toximul R and Toximul S (50:50). An aliquot of the solution was further diluted to a concentration of test compound of 1000 parts per million (ppm). The formulated test compound was sprayed onto the leaves of squash and bean plants, and the plants were then allowed to dry for two hours. All leaves were then removed from the treated plants, and the plants were placed in a greenhouse. Evaluations for terminal growth and morphological effects were made after five days. Growth regulator activity is rated on a scale of +3 to -3. A "+" indicates growth promotion, while a "-" indicates growth inhibition. The numerical ratings are assigned the meanings:

0 = no effect
1 = slight response
2 = moderate response
3 = distinct response.

Phytotoxicity is indicated according to the following scale:

B = burn
C = chlorosis
X = death

The results of this plant growth regulator test are presented in Table 13 which follows:

Table 13

Plant Growth Regulator Activity

| Compound of Example No. | Concentration ppm | Squash | | Bean | |
|---|---|---|---|---|---|
| | | Growth Rating | Phyto-toxicity | Growth Rating | Phyto-Toxicity |
| 1 | 1000 | 0 | 2C | -3 | 3C |
| 2 | 1000 | -1 | 1C | 3X | -3B |
| 3 | 1000 | | | -3 | 0CX |
| 4 | 1000 | -3 | 1C | 3X | 3X |
| 5 | 1000 | | | -1 | 0C |
| 8 | 1000 | -1 | 0C | -3B | -3B |
| 10 | 1000 | 0 | 0 | 0 | 0 |
| 11 | 1000 | | | -1 | 1C |

The compounds provided by this invention also have demonstrated useful activity as aquatic algicides, aquatic growth regulators and aquatic herbicides. A further embodiment of this invention therefore is a method for controlling aquatic plant growth which involves applying an effective amount of a compound as defined herein to the aquatic plants to be controlled or to the water in which the plants are growing.

The compounds of the invention have been evaluated in a standard aquatic algicide test designed to show algicidal activity. In a primary screen, the compounds were evaluated against *Chlorella vulgaris*, *Scenedesmus quadricanda*, and *Anacystis nidulans*. These algae species were grown on agar slants containing artificial media (Hughes' media). The agar slants were employed in the inoculation of the test media, which itself is an aqueous Hughes' media. Five milliliters of sterile Hughes' media is used to wash the agar slants, and this is then added to 400 ml. of sterile Hughes' media. Two milliliters of the inoculated media is then added to each of several 12 ml. disposable vials.

The test compounds are formulated for evaluation by dissolving 10 mg. of compound in a solution of 0.5 ml. acetone and 4.5 ml. of sterile 0.1% aqueous polyoxyethylene sorbitan monooleate (Tween 80). Aliquot portions of the formulated test compounds are then added to the vials containing the various algae species. Visual observations and comparisons to non-treated control vials were made 7 days after treatment. Activity ratings were made on a scale of 1 to 5 according to the following meanings:

1 = no effect
2 = slight effect
3 = moderate effect
4 = heavy effect
5 = 100% control.

Table 14 which follows presents the algicidal activity of compounds of the invention evaluated according to the foregoing procedure.

Table 14

Aquatic Algicide Activity

| Compound of Example No. | Concentration ppm | Control Ratings: Chlorella vulgaris | Control Ratings: Scenedesmus quadricanda | Control Ratings: Anacystis nidulans |
|---|---|---|---|---|
| Control | | 0 | 0 | 0 |
| 1 | 10 | 4 | 5 | 5 |
| | 1 | 3 | 3 | 5 |
| | 0.5 | 3 | 3 | 5 |
| 2 | 10 | 5 | 5 | 4 |
| | 1 | 1 | 1 | 1 |
| | 0.5 | 1 | 1 | 1 |
| 3 | 10 | 5 | 5 | 5 |
| | 1 | 4 | 5 | 4 |
| | 0.5 | 4 | 5 | 5 |
| 4 | 10 | 5 | 5 | 5 |
| | 1 | 2 | 2 | 2 |
| | 0.5 | 2 | 2 | 3 |
| 5 | 10 | 1 | 1 | 1 |
| 6 | 10 | 4 | 4 | 4 |
| | 1 | 5 | 4 | 1 |
| | 0.5 | 5 | 4 | 1 |
| 8 | 10 | 2 | 4 | 5 |
| 10 | 10 | 5 | 5 | 5 |
| | 1 | 5 | 5 | 5 |
| | 0.5 | 5 | 5 | 1 |
| 11 | 10 | 1 | 1 | 1 |

Several of the compounds provided by the invention were evaluated in a secondary aquatic algicide screen. The test was carried out as described above, except that several additional species of algae were employed. The results of the secondary screen are reported in Table 15 which follows.

Table 15

Aquatic Algicide Activity

| Compound of Example No. | Concentration ppm | Chlorella vulgaris | Scenedesmus quadricanda | Anacysts nidulans | Chlamydomonas moewus |
|---|---|---|---|---|---|
| Control | | 0 | 0 | 0 | 0 |
| 1 | 0.5 | 5 | 5 | 5 | 5 |
| | 0.1 | 5 | 5 | 4 | 1 |
| | 0.05 | 4 | 5 | 1 | 1 |
| 4 | 0.5 | 3 | 4 | 1 | 1 |
| | 0.1 | 1 | 1 | 1 | 1 |
| | 0.05 | 1 | 1 | 1 | 1 |
| 9 | 0.5 | 3 | 2 | 1 | 1 |
| | 0.1 | 1 | 1 | 1 | 1 |
| | 0.05 | 1 | 1 | 1 | 1 |
| 10 | 0.5 | 5 | 4 | 5 | 5 |
| | 0.1 | 5 | 4 | 3 | 4 |
| | 0.05 | 1 | 4 | 1 | 3 |

Table 15 continued

Aquatic Algicide Activity

| Compound of Example No. | Concentration ppm | Anabaena flos-aqua | Anabaena blue green | Anabaena 1552 | Oscillatori 1270 |
|---|---|---|---|---|---|
| Control | | 0 | 0 | 0 | 0 |
| 1 | 0.5 | 5 | 5 | 5 | 4 |
| | 0.1 | 4 | 1 | 3 | 3 |
| | 0.05 | 4 | 1 | 2 | 3 |
| 4 | 0.5 | 1 | 1 | 1 | 1 |
| | 0.1 | 1 | 1 | 1 | 1 |
| | 0.05 | 1 | 1 | 1 | 1 |
| 9 | 0.5 | 1 | 2 | 1 | 1 |
| | 0.1 | 1 | 1 | 1 | 1 |
| | 0.05 | 1 | 1 | 1 | 1 |
| 10 | 0.5 | 5 | 5 | 5 | 5 |
| | 0.1 | 5 | 1 | 3 | 4 |
| | 0.05 | 5 | 1 | 3 | 2 |

The compound of Example No. 4 was further evaluated as an aquatic algicide by direct comparison with the known algicides cutrine-plus (a mixed copper-ethanolamine complex) and aquazine (simazine, 2-chloro-4,6-bis(ethylamino)-s-triazine). The compounds were compared by evaluation against Anabaena sp. according to the general test procedure outlined above. Table 16 which follows presents the relative activities of the compounds.

Table 16

Algicide Activity on Anabaena sp.

| | Rate (ppm) | % Control one week post treatment |
|---|---|---|
| Compound of Example 4 | 1.0 | 67 |
| | 0.3 | 63 |
| | 0.1 | 60 |
| Cutrine-Plus | 0.4 | 60 |
| Aquazine Control | 1.6 | 53 |

As noted above, the compounds of this invention also have activity in the regulation of aquatic plant growth and are useful as aquatic herbicides. The following method was used in the laboratory to evaluate the aquatic growth regulating properties of the compounds disclosed herein.

The compounds for this test were formulated in the following manner. Twenty milligrams of compound was weighed into a 12 ml. disposable vial. To the vial containing the compound were added 1 ml. of acetone and 9 ml. of aqueous 0.1 percent polyoxyethylene sorbitan

monooleate (Tween 80). This solution was then diluted with appropriate volumes of water to obtain solutions containing 10, 1, 0.5 and 0.25 ppm (parts per million) of test compound.

Terminal pieces of Florida elodea, *Hydrilla verticillata* (L.F.), (hereinafter identified as hydrilla) 10 cm. long, without branching, were prepared for testing. Three such cuttings were placed in each plastic container holding 785 ml. of water containing the formulated test compound and 3 ml. of Hoagland's nutrient solution. Three 10 cm. cuttings of hydrilla were placed in each of several control containers of water. To the water in each control container there was also added the amount of solvent used to formulate the test compound for each container.

After a period of two to three weeks, measurements were made to determine the total length of each plant. An average total growth was obtained by dividing the total combined lengths by the number of replicates. By subtracting 10 cm. from the average total length, the average increase in growth was obtained. This difference was divided by the average increase in length of the plants in the solvent controls (SC) and the quotient multiplied by 100 to give a percent inhibition.

$$\frac{\text{Total combined length of Replicates}}{\text{Number of Replicates}} = \text{Average Length}$$

$$\text{Avg. Length} - 10\text{ cm.} = \text{Avg. Increased Growth}$$

$$\frac{\text{Avg. Increased Growth}}{\text{Avg. Increased Growth SC}} \times 100 = \%\ \text{Inhibition}$$

Each compound employed in this experiment, is identified by the number of the operating example describing its preparation.

The results of the tests, run at the reported concentration levels in ppm. of compound, and observed at the end of three weeks, are set forth in Table 17, which follows.

Table 17

Aquatic Growth Regulator Activity

| Compound of Example No. | Approximate % Growth Inhibition at Indicated Test Concentrations | | | |
|---|---|---|---|---|
| | 10 ppm | 1 ppm | 0.5 ppm | 0.25 ppm |
| 2 | 73 | -2 | 13 | 14 |
| 3 | 100 | 86 | 91 | 87 |
| 4 | 100 | 80 | 74 | 76 |
| 5 | 16 | | | |
| 6 | 100 | 78 | 59 | 48 |
| | 59 | 67 | 53 | 36 |
| 8 | 75 | 2 | 16 | 12 |

Several of the compounds of the invention were evaluated further as aquatic herbicides by employing additional weed species. The compounds were formulated for evaluation as described above, and the herbicidal activity was determined by visual observations based upon non-treated controls. Activity ratings were made on a scale of 1 to 5 as follows:

1 = no observable effect
2 = slight plant injury
3 = moderate plant injury
4 = 50-90% plant injury
5 = plants completely killed

The results of this test are reported in Tables 18 and 19 which follow.

Table 18

Aquatic Herbicide Activity 1-week post-treatment

| Compound of Example No. | Concentration ppm | Hydrilla | Coontail | Duckweed |
|---|---|---|---|---|
| Control | | 1 | 1 | 1 |
| 1 | 10 | 5 | 5 | 2 |
| | 4 | 1 | 1 | 1 |
| | 2 | 1 | 1 | 1 |
| 2 | 10 | 3 | 3 | 1 |
| 3 | 10 | 1 | 1 | 1 |
| 4 | 10 | 1 | 1 | 1 |
| 5 | 10 | 1 | 1 | 1 |
| 6 | 10 | 1 | 1 | 1 |
| 8 | 10 | 1 | 1 | 1 |
| 10 | 4 | 4 | - | 4 |
| | 2 | 4 | - | 4 |
| 11 | 10 | 1 | 1 | 1 |

Table 19

Aquatic Herbicide Activity

| Compound of Example No. | Concentration ppm | 1-week post treatment | | | | | | | 2-week post treatment | | | | | | | 3-week post treatment | | | | | | |
|---|---|---|---|---|---|---|---|---|---|---|---|---|---|---|---|---|---|---|---|---|---|---|
| | | Hydrilla | Coontail | Duckweed | Naiad | Milfoil | Cabomba | Chara | Hydrilla | Coontail | Duckweed | Naiad | Milfoil | Cabomba | Chara | Hydrilla | Coontail | Duckweek | Naiad | Milfoil | Cabomba | Chara |
| Control | | 1 | 1 | 1 | 1 | 1 | 1 | 1 | 1 | 1 | 1 | 1 | 1 | 1 | 1 | 1 | 1 | 1 | 1 | 1 | 1 | 1 |
| 1 | 2 | 3 | 2 | 2 | 4 | 2 | 3 | 2 | 3 | 4 | 4 | 5 | 5 | 5 | 2 | 4 | 4 | 4 | 5 | 5 | 5 | 1 |
| | 1 | 3 | 2 | 2 | 4 | 1 | 3 | 3 | 5 | 4 | 3 | 5 | 5 | 5 | 5 | 5 | 4 | 4 | 5 | 5 | 5 | 4 |
| | 0.5 | 1 | 2 | 2 | 4 | 2 | 3 | 1 | 2 | 4 | 3 | 5 | 4 | 4 | 2 | 5 | 4 | 4 | 5 | 5 | 5 | 1 |
| 4 | 2 | 1 | 2 | 2 | 2 | 1 | 3 | 1 | 1 | 1 | 3 | 5 | 2 | 3 | 2 | 2 | 2 | 4 | 5 | 4 | 4 | 1 |
| | 1 | 1 | 2 | 2 | 1 | 1 | 1 | 1 | 3 | 4 | 3 | 4 | 3 | 4 | 1 | 3 | 4 | 4 | 5 | 5 | 5 | 4 |
| | 0.5 | 1 | 2 | 2 | 1 | 1 | 2 | 1 | 1 | 1 | 2 | 1 | 3 | 3 | 2 | 3 | 5 | 4 | 4 | 4 | 5 | 4 |
| 9 | 2 | 2 | 3 | 3 | 2 | 1 | 2 | 2 | 2 | 4 | 2 | 3 | 1 | 4 | 2 | 3 | 4 | 3 | 3 | 4 | 5 | 3 |
| | 1 | 1 | 3 | 1 | 1 | 2 | 3 | 1 | 2 | 4 | 2 | 1 | 2 | 3 | 1 | 3 | 4 | 2 | 1 | 5 | 4 | 1 |
| | 0.5 | 2 | 4 | 1 | 1 | 3 | 3 | 1 | 2 | 4 | 1 | 1 | 2 | 2 | 1 | 2 | 4 | 1 | 1 | 3 | 2 | 1 |
| 10 | 2 | 2 | 2 | 1 | 4 | 3 | 3 | 1 | 5 | 5 | 3 | 5 | 5 | 5 | 3 | 5 | 5 | 4 | 5 | 5 | 5 | 3 |
| | 1 | 3 | 2 | 2 | 3 | 1 | 3 | 1 | 5 | 5 | 4 | 5 | 5 | 5 | 5 | 5 | 5 | 4 | 5 | 5 | 5 | 5 |
| | 0.5 | 3 | 3 | 1 | 3 | 1 | 3 | 1 | 5 | 4 | 3 | 5 | 5 | 5 | 5 | 5 | 5 | 4 | 5 | 5 | 5 | 5 |

The compounds of the invention are also useful in the control of water hyacinth. Water hyacinth is a floating aquatic plant of the order Eichhornia crassipes of the family Pontederiaceae. The plant is very troublesome in warm regions since it floats on water and often clogs waterways, and is very difficult to control safely.

Several of the compounds provided herein have been evaluated for their ability to control the growth of water hyacinth. In one such study, the test compounds were formulated in acetone and Tween 80 as described above and sprayed onto the leaves of water hyacinth plants which were growing in buckets placed in a greenhouse. The herbicidal activity was then determined by visual inspection seven days following application. Observations were made of treated plants and non-treated control plants. The activity of the compounds was also compared to that of the commercial herbicide diuron, a dichlorophenylurea derivative. The results of the test are presented in Table 20.

Table 20

Activity against Water Hyacinth

% Control

| Compound of Example No. | Rate lb/A | Rate kg/ha | Foliar Application 1-week post treatment |
|---|---|---|---|
| Control | 0 | | 0 |
| 1 | 0.25 | 0.28 | 98 |
| | 0.08 | 0.09 | 96 |
| | 0.025 | 0.028 | 58 |
| 4 | 0.25 | 0.28 | 75 |
| | 0.08 | 0.09 | 28 |
| | 0.025 | 0.028 | 23 |
| 9 | 0.25 | 0.28 | 77 |
| | 0.08 | 0.09 | 37 |
| | 0.025 | 0.028 | 3 |
| 10 | 0.25 | 0.28 | 99 |
| | 0.08 | 0.09 | 91 |
| | 0.025 | 0.028 | 58 |
| Diuron | 0.25 | 0.28 | 81 |
| | 0.08 | 0.09 | 40 |
| | 0.025 | 0.028 | 10 |

The compound of Example 10 was further evaluated as a herbicide against water hyacinth when applied to the roots or to the leaves. The compound was formulated as before and applied to water hyacinth plants which were growing in jars placed in a greenhouse. Visual observations of efficacy were made seven days post application and again fourteen days post application. The results of the study are presented in Table 21.

Table 21

Activity Against Water Hyacinth

% Control

| Compound of Example No. | Rate PPM | Foliar Application | | Root Application | |
|---|---|---|---|---|---|
| | | 1-week post application | 2-weeks post application | 1-week post application | 2-weeks post application |
| Solvent Control | 0 | 0 | 0 | 0 | 0 |
| Diuron | 0.3 | 70 | 100 | 27 | 98 |
| | 0.1 | 50 | 43 | 17 | 15 |
| | 0.03 | 20 | 10 | 3 | 0 |
| 10 | 0.3 | 90 | 100 | 63 | 100 |

As the data presented in Tables 12-19 demonstrate, the compounds defined herein are useful in the control of aquatic vegetative growth. A further embodiment of this invention is therefore a method of eliminating and controlling the growth of aquatic plants. This method is practiced by adding the active isoxazolylimidazolidinone derivatives to the water containing the submerged or floating aquatic plants, or otherwise contacting the plants with the active compounds, for example, by applying the compounds to the sub-aqueous soil in which the aquatic plants are rooted. The compounds may be applied to the water as dusts when admixed with a powdered solid carrier such as bentonite, Fuller's earth, diatomaceous earth, or various mineral silicates, e.g., mica, talc, pyrophyllite, and clays. The compounds may also be mixed with surface-active dispersing agents to form concentrates to facilitate dispersion in water and to improve the wetting properties when used as sprays. If desired, the compounds may be mixed with a powdered solid carrier, together with a surface-active dispersing agent, so that a wettable powder may be obtained which may be applied directly, or which may be shaken with water to make an aqueous dispersion for application in that form. These wettable powder formulations suitably contain from about 25 to about 85 percent by weight of the active ingredient, i.e., an aquatic growth regulating compound coming within the scope of the generic formulae, <u>supra</u>. The compounds may be dissolved in an oil, such as a hydrocarbon or chlorinated hydrocarbon oil, and the oil solution of the compound dispersed in water with the

aid of a surface-active dispersing agent to give a sprayable aqueous dispersion. Such surface-active dispersing agents may be anionic, nonionic, or cationic surface-active agents. Such surface-active agents are well-known, and reference is made to Hoffman *et al.*, U.S. Patent No. 2,614,916, columns 2-4, for detailed examples of the same. The compounds useful in this embodiment of the invention may also be applied by the aerosol method. Solutions for the aerosol treatment may be prepared by dissolving the compound directly in the aerosol carrier, which is a liquid under pressure, but which is a gas at ordinary temperature (e.g. 20°C.) and atmospheric pressure; or, the aerosol solution may be prepared by first dissolving the compound in a less volatile solvent, and then admixing such solution with the highly volatile liquid aerosol carrier.

Further, the compounds useful as aquatic growth regulators can also be applied in an invert emulsion formulation. An invert emulsion formulation is prepared by first making a solution of an aquatic growth regulating compound in heavy oils, such as diesel fuel, inverting oil, and the like, and combining the thus-obtained solution with water under high shear stirring. The thick emulsion is placed in the water and sinks to the bottom of the lake, river, pond, or the like, and the aquatic growth regulator is gradually released to control the growth of the aquatic plants. The following is an example of an invert emulsion formulation, prepared using the compound of Example No. 1 of this application.

Invert Emulsion

| | |
|---|---|
| Compound of Example No. 1 | 12.5 gm |
| Diesel fuel | 333 ml |
| Inverting oil* | 333 ml |

*Vioko-Rhap Inverting Oil (Rhodia, Inc.)

Two-hundred fifty milliliters of this solution is combined with 3750 ml. of water under high shear stirring to give a thick invert emulsion.

The compounds useful as aquatic growth regulators can also be applied as pellets which are prepared from a mixture of about 5% of the active ingredient, about 85% clay, and about 10% water, all percentages being by weight. The mixture is then extruded through a pellet mill using a suitably sized die, e.g., about 1/8 in. diameter. The extruded pellets are about 1/8 in. by 1 1/2 in., and are then dried to about 8% moisture content.

The method of controlling aquatic plant growth provided by this invention is practiced by adding to the water containing the submerged or floating plants a growth-regulating or herbicidal amount of one of the herein-disclosed compounds, such that a concentration of from about 0.01 to about 10 ppm. of the active compound is attained. A preferred method of aquatic plant growth regulation provided by this invention is directed toward the control of plants such as water hyacinth. Such plants can be controlled by foliar or root application of a compound of this invention at a rate of about 0.01 to about 1.0 pounds per acre (about 0.011 to about 1.1 kg/ha).

The optimum concentration of active compound for any specific aquatic weed control problem varies with the temperature, the species to be controlled, and the shape of the body of water to be treated. At higher water temperatures, less compound is generally required for a given degree of control than is needed at lower temperatures. When used to control algae or aquatic plant growth, the compounds will usually be employed at concentrations of about 0.1 to about 10 ppm. In terms of pounds of compound per acre of water one foot deep, 0.1 to 10 ppm. is equal to about 0.3 to about 30 pounds per acre of water one foot deep.

A preferred aquatic plant growth regulation method according to this invention comprises employing the compound of Example Nos. 1, 2, 4, 9, and 10. Such compounds may be applied to the aqueous growth environments as dispersible powders or in solution with water-miscible solvents.

Claims

1. An isoxazolylimidazolidinone of the formula I

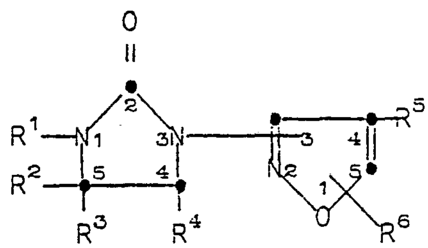

I

wherein:

$R^1$ is $C_1$-$C_6$ alkyl;

$R^2$ is hydrogen, or taken together with $R^4$ is a double bond;

$R^3$ is hydrogen or $C_1$-$C_6$ alkyl;

$R^4$ is hydroxy, $C_1$-$C_6$ alkanoyloxy, or taken together with $R^2$ is a double bond;

$R^5$ is hydrogen, halo, cyano, nitro, or $C_1$-$C_6$ alkyl; and

$R^6$ is hydrogen, $C_1$-$C_6$ alkyl, trifluoromethyl, $C_1$-$C_3$ alkoxy-$C_1$-$C_6$ alkyl, $C_3$-$C_7$ cycloalkyl or $C_1$-$C_3$ alkyl substituted $C_3$-$C_7$ cycloalkyl.

2. An isoxazolylimidazolidinone of the formula I selected from:

3-(5-tert.-butyl-3-isoxazolyl)-4-hydroxy-1-methyl-2-imidazolidinone;

3-(5-methyl-3-isoxazolyl)-4-hydroxy-1-methyl-2-imidazolidinone;

3-(5-isopropyl-3-isoxazolyl)-4-hydroxy-1-methyl-2-imidazolidinone;

3-(3-tert.-butyl-5-isoxazolyl)-4-hydroxy-1-methyl-2-imidazolidinone;

3-(3-methyl-5-isoxazolyl)-4-hydroxy-1-methyl-2-imidazolidinone;

3-(3-isopropyl-5-isoxazolyl)-4-hydroxy-1-methyl-2-imidazolidinone;

3-(3-(1,1-dimethyl-2-methoxyethyl)-5-isoxazolyl)-4-hydroxy-1-methyl-2-imidazolidinone;

3-(3-methyl-4-cyano-5-isoxazolyl)-4-hydroxy-1-methyl-2-imidazolidinone;

3-(3-<u>tert</u>.-butyl-5-isoxazolyl)-4-acetoxy-1-methyl-2-imidazolidinone;

3-(5-<u>tert</u>.-butyl-3-isoxazolyl)-4-acetoxy-1-methyl-2-imidazolidinone;

3-(3,4-dimethyl-5-isoxazolyl)-1-methyl-2-imidazolinone;

3-[5-(1-ethyl-1-methylpropyl)-3-isoxazolyl]-4-hydroxy-1-methyl-2-imidazolidinone;

3-[5-(1-ethyl-1-methylpropyl)-3-isoxazolyl]-4-acetoxy-1-methyl-2-imidazolidinone;

3-[5-(1-ethylcyclohexyl)-3-isoxazolyl]-4-hydroxy-1-methyl-2-imidazolidinone;

3-[5-(1-ethylcyclohexyl)-3-isoxazolyl]-4-acetoxy-1-methyl-2-imidazolidinone;

3-(5-n-propyl-3-isoxazolyl)-4-hydroxy-1-methyl-2-imidazolidinone; and

3-(5-n-propyl-3-isoxazolyl)-4-acetoxy-1-methyl-2-imidazolidinone.

3. A herbicidal formulation which comprises as active ingredient from 1.0 to 90.0% by weight of an isoxazolylimidazolidinone of the formula I as claimed in claim 1 or 2 associated with at least 1 carrier or diluent therefor.

## Claims

1. A process for preparing an isoxazolylimidazolidinone of the formula I

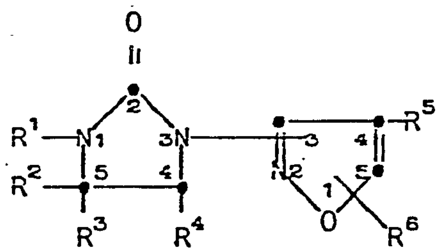

I

wherein:

$R^1$ is $C_1$-$C_6$ alkyl;

$R^2$ is hydrogen, or taken together with $R^4$ is a double bond;

$R^3$ is hydrogen or $C_1$-$C_6$ alkyl;

$R^4$ is hydroxy, $C_1$-$C_6$ alkanoyloxy, or taken together with $R^2$ is a double bond;

$R^5$ is hydrogen, halo, cyano, nitro, or $C_1$-$C_6$ alkyl; and

$R^6$ is hydrogen, $C_1$-$C_6$ alkyl, trifluoromethyl, $C_1$-$C_3$ alkoxy-$C_1$-$C_6$ alkyl, $C_3$-$C_7$ cycloalkyl or $C_1$-$C_3$ alkyl substituted $C_3$-$C_7$ cycloalkyl, which comprises cyclising α (dialkoxy)ethyl isoxazolylurea of the formula II

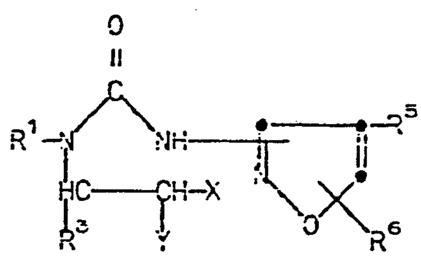

II

wherein $R^1$, $R^3$, $R^5$ and $R^6$ are as above described and X and Y independently are $C_1$-$C_6$ alkoxy

to obtain a compound of formula I in which $R^4$ is hydroxy and, optionally, either dehydrating the isoxazolylimidazolidinone of the formula I when $R^4$ is hydroxy to obtain a compound of formula I wherein $R^4$ together with $R^2$ signifiy a double bond or acylating the isoxazolylimidozolidinone of the formula I when $R^4$ is hydroxy to obtain a compound of formula I wherein $R^4$ is $C_1$-$C_6$ alkanoyloxy

2. An isoxazolylidinone of the formula I whenever prepared by a process according to claim 2.

3. A method of inhibiting the growth of weeds which comprises applying to the locus of weeds a herbicidally effective amount of an isoxazolylimidazolidinone of formula I as according to claim 1.

4. A method of inhibiting the growth of weeds as claimed in claim 3 which comprises applying to the locus of terrestrial weeds a herbicidally effective amount of an isoxazolylimidazolidinone of formula I.

5. A method of inhibiting the growth of weeds as claimed in claim 3 which comprises applying to the locus of aquatic weeds a herbicidally effective amount of an isoxazolylimidazolidinone of formula I.

6. A method of inhibiting the growth of weeds as claimed in claim 3 which comprises applying to the locus of water hyacinth a herbicidally effective amount of an isoxazolylimidazolidinone of formula I.

7. A method of producing a herbicidal composition which comprises admixing a compound of formula I, stated in claim 1, with an agriculturally acceptable diluent or carrier.